# EUROPEAN PATENT APPLICATION

(11) **EP 4 681 738 A1**
(43) Date of publication of application: **21.01.2026**
(21) Application number: 23927043.2
(22) Date of filing: 31.08.2023
(51) Int. Cl.: A61K 47/32, A61P 9/10, A61P 9/00, A61P 3/10, A61P 3/06, A61P 1/16, A61P 1/00, A61P 29/00, A61P 15/00, A61P 3/04, A61P 25/28

(54) **1,4-POLYISOPRENE DISPERSION SYSTEM, PHARMACEUTICAL ACTIVE INGREDIENT AND USE THEREOF**

(30) Priority: 14.03.2023 CN 202310243245; 25.06.2023 CN 202310751990; 04.08.2023 CN 202310978602
(71) Applicant: Suzhou Haiyi Biomedical Technology Co., Ltd, Suzhou, Jiangsu 215421 (CN)
(72) Inventor: ZHU, Yixin, Suzhou, Jiangsu 215000 (CN); CHEN, Yuanli, Suzhou, Jiangsu 215000 (CN); YANG, Xiaoxiao, Suzhou, Jiangsu 215000 (CN); XU, Shuang, Suzhou, Jiangsu 215000 (CN)
(74) Representative: Sonnenhauser, Thomas Martin
(86) International application number: PCT/CN2023/116063
(87) International publication number: WO 2024/187697

(57) **Abstract**

A 1,4-polyisoprene dispersion system, pharmaceutical active ingredient and the use thereof. The dispersion system is stable in the gastric acid environment of mammals without precipitation of 1,4-polyisoprene clots, has no oral toxicity to mammals, and does not contain allergens. The 1,4-polyisoprene dispersion system comprises a 1,4-polyisoprene solution dispersion system and a 1,4-polyisoprene emulsion dispersion system. The 1,4-polyisoprene dispersion system can serve as a pharmaceutical active ingredient to be used for preparing drugs for treating diseases including atherosclerotic cardiovascular and cerebrovascular diseases, type II diabetes, hypercholesterolemia, hypertriglyceridemia, fatty liver, colitis, obesity, polycystic ovary syndrome and Alzheimer's disease.

## Description

### FIELD OF THE INVENTION

The application belongs to the technical field of pharmaceutical material, specifically relates to a 1,4-polyisoprene dispersion system and pharmaceutical active ingredient and use thereof.

### BACKGROUND

The chronic metabolic disease is a group of metabolic clinical symptoms mainly comprising atherosclerotic cardiovascular and cerebrovascular diseases (coronary heart disease, cerebral apoplexy), type II diabetes, hypercholesterolemia, hypertriglyceridemia, fatty liver, chronic enteritis, polycystic ovary syndrome and adiposity; Although the fatality rate of diseases such as obesity, hyperlipidemia, fatty liver disease, polycystic ovary syndrome (PCOS), chronic enteritis is not as high as that of cardiovascular and cerebrovascular diseases or diabetesobesity, the incidence rate is high in adults, the number of patients is large, and the health hazard is great.

In the existing technology for treating chronic metabolic disease, people respectively develop many drugs aiming at different diseases. Taking atherosclerotic heart disease as an example, the existing medicaments include lipid-lowering medicaments for stabilizing atherosclerotic plaques such as statins and PCSK9 inhibitors, antiplatelet medicaments such as aspirin, clopidogrel, blood pressure lowering medicaments such as beta receptor blockers, RAS inhibitors and so on.

In the existing art, there is also ongoing research and development of drugs targeting various conditions of chronic metabolic diseases. CN101505594A discloses a drug that can be used to treat a range of metabolic disorders, such as insulin resistance syndrome, diabetes, polycystic ovary syndrome, hyperlipidemia, fatty liver disease, cachexia, obesity, atherosclerosis, and arteriosclerosis.

Despite this, chronic metabolic diseases remain a category of illnesses with high mortality rates, a large patient population, and severe harm to human health. There is a need to develop more and better drugs to address the challenges posed by these diseases.

### SUMMARY

The invention provide a 1,4-polyisoprene dispersion system and pharmaceutical active ingredient and use thereof. The invention uses the 1,4-polyisoprene dispersion system as a pharmaceutical active ingredient. Because of its characteristics of remaining stable in the mammalian gastric acid environment, with no formation of 1,4-polyisoprene coagulum, and exhibiting no oral toxicity or being free from allergens, it is used in the manufacture of a medicament for treating chronic metabolic diseases, including, but not limited to, atherosclerotic cardiovascular and cerebrovascular diseases, type II diabetes mellitus, hypercholesterolemia, hypertriglyceridemia, fatty liver disease, colitis, obesity, and polycystic ovary syndrome (PCOS).

In a first aspect, the present application provides a 1,4-polyisoprene dispersion system that remains stable in a gastric acid environment in a mammal without the precipitation of 1,4-polyisoprene clot, absence of oral toxicity and being free from allergens .

In the present application, the 1,4-polyisoprene in the 1,4-polyisoprene dispersion system is present in a dispersed state. The application uses the1,4-polyisoprene dispersion system as a pharmaceutical active ingredient, which remains stable in a gastric acid environment in a mammal without the precipitation of 1,4-polyisoprene clot, and has no oral toxicity to the mammal and is free from allergens, It is used for preparing medicament for treating chronic metabolic diseases including atherosclerosis cardiovascular and cerebrovascular diseases, type II diabetes, hypercholesterolemia, hypertriglyceridemia, fatty liver, colitis, obesity and polycystic ovary syndrome.

The following is the preferred technical solution of the present application, but not the limitation of the technical solution provided by the present application, the purpose and beneficial effects of the present application can be better achieved through the following preferred technical solution.

As a preferred technical solution of the present application, the1,4-polyisoprene dispersion system is selected from a 1,4-polyisoprene solution dispersion system and a 1,4-polyisoprene emulsion dispersion system,
wherein the 1,4-polyisoprene solution dispersion comprises 1,4-polyisoprene and a solvent,
the 1,4-polyisoprene emulsion dispersion system comprises 1,4-polyisoprene, an emulsifier and water.

As a preferred technical solution of the present application, the polymerization degree of the 1,4-polyisoprene is ≥6, without upper limit, for example, 6, 10, 20, 50, 100, 200, 500, 1000, 2000, 5000, 8000, 10000 or 100000 and so on.

Because the 1,4-polyisoprene with polymerization degree less than 6 is volatile, and odorous, and has inhibiting effect on the cell growth cultured in vitro, the 1,4-polyisoprene with polymerization degree more than 6 is selected to prepare the1,4-polyisoprene dispersion system .

It should be noted that, in the present application, there is no special limitation on the cis-and trans-structure and source of 1,4-polyisoprene, which can be prepared from natural latex, and also can be obtained by artificial synthesis. The artificially synthesized 1,4-polyisoprene contains cis-and trans-structures at the same time, mainly cis-structure. In natural rubber, most plants, including Brazil three-leaf rubber tree, or fungal sources are cis structures. It is now clearly known that the natural rubber produced from the eucommia bark is trans-structure, i.e. trans-1,4-polyisoprene.

As a preferred technical solution of the present application, the solvent is selected from any one or a combination of at least two of fatty acids, fatty alcohols, esters or lipoid compounds.

Preferably, the ester compounds are selected from any one or a combination of at least two of monohydric alcohol ester, dihydric alcohol ester or polyhydric alcohol ester.

In the application, the monohydric alcohol ester is obtained by reaction of fatty acid and monohydric alcohol, the dihydric alcohol ester is obtained by reaction of fatty acid and monohydric alcohol, the polyhydric alcohol ester is obtained by reaction of fatty acid and polyhydric alcohol.

Preferably, the monohydric alcohol ester comprises fatty acid ethyl ester.

Preferably, the fatty acid ethyl ester is selected from any one or a combination of at least two of ethyl laurate, ethyl palmitate, ethyl stearate, ethyl oleate, ethyl linoleate, ethyl linoleate, eicosapentaenoic acid (EPA) ethyl ester or docosahexaenoic acid (DHA) ethyl ester.

Preferably, the polyol ester comprises a glycerol ester.

Preferably, the glycerol ester is selected from any one or a combination of at least two of monoglycerides, diglycerides or triglycerides.

Preferably, the monoglycerides and diglycerides are obtained by reacting lauric acid, palmitic acid, stearic acid, oleic acid, linoleic acid, linolenic acid with glycerol.

Preferably, the triglycerides include synthetic C6-C12 medium-chain fatty acid triglycerides and/or natural triglycerides such as coconut oil.

Preferably, the lipid compound is selected from any one or a combination of at least two of squalene, phospholipid, glycolipid, sphingolipid, steroid or squalene.

Preferably, the lipid compound comprises squalene.

It should be noted that the selection of solvents for the 1,4-polyisoprene solution dispersion system described in this application is primarily based on considerations such as favorable safety profiles, high solubility for 1,4-polyisoprene, and low viscosity.

In the monohydric alcohol ester, the safety of the ethanol ester is better than that of the methanol ester. Hence, the preferred choice for the monohydric alcohol is ethyl ester. In the ethanol ester, if the number of carbon atoms in the carbon chain of the fatty acid is less than 12, the solubility of the 1,4-polyisoprene is reduced, so the fatty acid with the number of carbon atoms more than or equal to 12 is selected; The higher the degree of unsaturation in fatty acids having a carbon number of not less than 18, the better the flowability at room temperature. Thus, the monohydric alcohol ester is preferably any one or a combination of at least two of ethyl laurate, ethyl palmitate, ethyl stearate, ethyl oleate, ethyl linoleate, ethyl linolenate, eicosapentaenoic acid (EPA) ethyl ester, docosahexaenoic acid (DHA) ethyl ester.

In polyhydric alcohol ester, glycerol esters, including monoglycerides, diglycerides and triglycerides, are preferred for the safety of glycerol esters (glycerol esters). The monoglycerides and diglycerides produced by C12-C18 fatty acid and glycerol have small molecular weight, low viscosity and good solubility to 1,4-polyisoprene. Therefore, it is preferable that any one of lauric acid, palmitic acid, stearic acid, oleic acid, linoleic acid, and linolenic acid react with glycerol to obtain monoglycerides or diglycerides. The triglyceride has three ester bonds and three fatty acids. If the fatty acid is a long-chain fatty acid having more than 12 carbon atoms, the total molecular weight of the triglyceride is large, the viscosity is high, and the solubility of the fatty acid to the 1,4-polyisoprene is poor; On the contrary, if the fatty acid is a short chain fatty acid having less than 6 carbon atoms, the overall molecule of the triglyceride is strongly non-polar and has poor solubility for 1,4-polyisoprene. Therefore, the molecular weight of the medium chain fatty acid triglyceride having 6 to 12 carbon atoms (for example, 6,8, 10 or 12) is moderate, the viscosity is moderate, and the solubility to the 1,4-polyisoprene is good. The medium-chain fatty acid triglyceride having 6-12 carbon atoms comprises artificially synthesized medium-chain fatty acid glyceride and coconut oil in natural grease.

Preferably, the lipid compounds are selected from the group consistiong of phospholipids, glycolipids, sphingolipids, steroidal compounds, squalene, etc., most of which are solids at room temperature and exhibit poor solubility in 1,4-polyisoprene. Therefore, squalene, which is liquid at room temperature and, like 1,4-polyisoprene, is a non-polar molecule, is preferred as the solvent.

As a preferred technical solution of the present application, the mass percentage content of the 1,4-polyisoprene in the 1,4-polyisoprene solution dispersion system is less than or equal to 60 %, and is not 0 (for example, 0.05 %, 0.1 %, 0.5 %, 1 %, 2 %, 5 %, 7 %, 10 %, 12 %, 15 %, 18 %, 20 %, 30 %, 40 %, 50 % or 60 % and the like), preferably from 0.2 % to 20 %.

In the 1,4-polyisoprene solution dispersion system, as the mass percentage of 1,4-polyisoprene increases, the solution becomes increasingly viscous, making it difficult to be prepared and be used. The solvent with small molecular weight and high fluidity can reduce the viscosity of the solution to a certain extent. The study found that it was difficult to prepare a 1,4-polyisoprene solution dispersion system at a content of 60 % or more by the prior art, and that it had good preparation efficiency and flowability at a content of 20 % or less, and hence the upper limit was selected to be 60 %, more preferably 20 %. The content of 1,4-polyisoprene has no lower limit, but with the decrease of the content of 1,4-polyisoprene, in order to obtain the medicament activity with therapeutic effect, the mammal or cell must use a larger volume of solution, and the use convenience is decreased.

It should be noted that the preparation method of the 1,4-polyisoprene solution dispersion system in the present application is not limited to any particular method, and only a system in which the 1,4-polyisoprene molecules are completely dispersed in a solvent is required, and the method includes, but is not limited to, the following steps.

The bulk 1,4-polyisoprene derived from the rubber tree Hevea brasiliensis is frozen in liquid nitrogen to form a brittle solid, which is then pulverized using a high-speed crusher. The crushed particles are passed through a 20-mesh sieve and placed in a solvent with continuous stirring until the 1,4-polyisoprene is completely dissolved, resulting in the 1,4-polyisoprene solution dispersion system.

The process of crushing the 1,4-polyisoprene with a high-speed crusher and sieving the particles through a 20-mesh sieve is carried out in order to accelerate its dissolution in the solvent.

As a preferred technical solution of the present application, the emulsifier is selected from any one or a combination of at least two of cationic surfactant, anionic surfactant, ionic surfactant and nonionic surfactant.

Preferably, the nonionic surfactant is at least one selected from the group consisting of polyoxyethylene (20) sorbitan monolaurate (Tween-20), polyoxyethylene (20) sorbitan monopalmitate (Tween-40), polyoxyethylene (20) sorbitan monostearate (Tween-60), polyoxyethylene (20) sorbitan monooleate (Tween-80), polyglycerol monolaurate (Q-12S), polyglycerol monomyristate (Q-14S), polyglycerol monopalmitate (Q-16S), polyglycerol monooleate (Q-17S), polyglycerol monostearate (Q-18S) and C12-C18 mono-fatty acid sucrose ester.

It should be noted that, within the prior art, millions of surfactants with vastly different properties and a wide variety of types are already known. The preferred surfactants are merely those selected by the present application from common low-toxicity or non-toxic surfactants, which have been experimentally confirmed to maintain the stability of the 1,4-polyisoprene emulsion dispersion system in a strongly acidic environment at pH=1. There remains the possibility that other applicable emulsifiers (including a mixture of two or more emulsifiers ) have not been tested or identified as preferred, which could be discovered and selected through simple repeat testing. Emulsion dispersion systems comprising these potentially applicable emulsifiers and 1,4-polyisoprene also fall within the scope of protection of this patent.

As a preferred technical solution of the present application, the mass percentage content of 1,4-polyisoprene in the 1,4-polyisoprene emulsion system is less than or equal to 80 %, and is not 0 (for example, 0.05 %, 0.1 %, 0.5 %, 1 %, 2 %, 5 %, 10 %, 15 %, 20 %, 25 %, 30 %, 35 %, 40 %, 45 %, 50 %, 60 %, 70 % or 80 % and so on), further preferably 0.5 % to 70 %.

In the 1,4-polyisoprene emulsion dispersion system, a higher mass percentage of 1,4-polyisoprene results in poorer flowability and stability of the emulsion. A mass percentage of 80% represents the highest concentration of 1,4-polyisoprene that can be prepared and remain stable under the existing technical conditions described in this application, although its flowability is relatively poor. When the concentration is reduced to 70%, both the stability and flowability of the emulsion reach satisfactory levels. Therefore, the upper limit for the 1,4-polyisoprene content in the emulsion is set at 80%, with 70% being preferred. There is no defined lower limit for the 1,4-polyisoprene content. However, as the concentration decreases, a larger volume of the emulsion must be administered to mammals or cells to achieve the desired therapeutic effect, resulting in reduced convenience of use. Studies have found that a concentration of 0.5% represents the lower limit for acceptable usability. Therefore, 0.5% is preferred as the lower limit for the mass percentage of 1,4-polyisoprene.

As a preferred technical solution of the present application, the average particle diameter of the emulsion droplets in the 1,4-polyisoprene emulsion system is ≤10 µm, for example, 0.1 µm, 0.5 µm, 1 µm, 2 µm, 3 µm, 4 µm, 5 µm, 6 µm, 7 µm, 8 µm, 9 µ m or 10 µ m and so on.

The smaller the particle diameter of the 1,4-polyisoprene emulsion dispersion system, the better the dispersion effect. The research shows that when the average particle diameter of the emulsion liquid in the 1,4-polyisoprene emulsion system is ≤10 µ m, the obvious medicament effect can be obtained.

It should be noted that the 1,4-polyisoprene emulsion dispersion system which can be used as a pharmaceutical active ingredient in the present application and the natural latex selected from Brazil's three-leaf rubber tree in the prior art, industrial concentrated latex prepared by natural latex as raw material and artificially synthesized 1,4-polyisoprene latex are different materials, the components and properties are greatly different, and cannot be mixed and replaced.

The components and characteristics of the 1,4-polyisoprene emulsion dispersion system of the present application are as follows:
1) few and definite components, consisting of 1,4-polyisoprene, water and emulsifier;
2)it remains stable in a strongly acidic environment with a pH of 1, thereby ensuring it stays dispersed in the mammalian gastric acid environment without the precipitation of 1,4-polyisoprene clots.;
3) there is no allergen, which will not cause allergy of human body and other mammals;
4) the components are safe to the mammal without oral toxicity.

The components and characteristics of the natural latex are as follows:
1) The components are complex and not fully defined. In addition to containing 1,4-polyisoprene and water, it also includes five major categories of substances: proteins, lipids, water-soluble substances, acetone-soluble substances, and inorganic salts. Proteins account for 1%-2% of the fresh latex mass, with approximately one-fifth distributed on the surface of rubber particles, forming a protective layer for the rubber particles. About three-fifths are dispersed in the whey, while the remainder is found in the bottom layer of the latex. Besides major proteins such as rubber protein and α-globulin, the protein components also include a large number of enzymes, including known types like coagulase, oxidase, peroxidase, reductase, protease, and phospholipase, among others. Lipids make up about 1% of the composition and consist of complex compounds such as triglycerides, fatty acids, waxes, sterols, sterol esters, and phospholipids. Acetone-soluble substances account for approximately 1% and are primarily consisting of oleic acid, linoleic acid, stearic acid, sterols, and sterol esters. Water-soluble substances constitute 1%-2% of the composition, with main components including quebrachitol and inositol isomers, sucrose, glucose, galactose, fructose, and pentoses. The inorganic salts mainly comprises ions such as potassium, calcium, magnesium, copper, iron, phosphate, sodium, and small amounts of sulfate, chloride, aluminum, manganese, nickel, rubidium, among others, and account for 0.3% to 0.7% of the latex mass.
2) It exhibits alkalinity and remains relatively stable in alkaline environments, but coagulates and precipitates upon contact with weak acids due to the separation of 1,4-polyisoprene.
3)It contains the rubber protein hevein (hevl), whose amino acid sequence shows high homology with the lectin (UDA) from Urtica dioica in the Urticaceae family. This protein is highly allergenic and serves as a major allergen in rubber products.

The natural rubber latex, in addition to the obvious sensitization, has the greatest problem of its acid-to-coagulation nature and therefore cannot be used as a medicinally active ingredient.

The components and characteristics of the concentrated natural latex and the synthetic latex are as follows:
1) Components are complex and unclear. The concentrated latex, in addition to the complex components of the natural latex, is also added with various chemical grade stabilizers, such as ammonia, TMTD (tetramethylthiuram disulfide) /ZnO, some of which are also added with formaldehyde, sodium sulfite, hydroxylamine, hydrogen peroxide, sodium carbonate, urea, organic amine (such as methylamine), sodium pentachlorophenate and so on. Some low ammonia or low protein concentrated latexes are also added with 2,2'-thiobis (4,6-dichlorophenol), 1,4-dioxanone and derivatives thereof, Stmktol LB219, dihydroxypropyl dodecanoate (BeKa100), Polyethylene glycol monocetyl ether sodium sulfate (trade name Levenol WX), ethoxylated fatty alcohols, ethoxylated alkyl phenols, ethoxylated long chain alkylamines, N - (2-hydroxy) propyl-3-trimethylamine chlorinated chitosan, triazine derivatives, and the like. Synthetic latex is a polyisoprene latex polymerized from chemical-grade polyisoprene monomers under the action of catalysts and initiators. In addition to the addition of various stabilizers, there may also be residues of catalysts (such as neodymium trioxide, tri-n-butyl phosphate, methylaluminoxane, etc.), solvents (which are usually toxic to some extent, such as toluene and n-hexane), monomers and oligomers (polyisoprene with very low polymerization degree), as well as approximately 5-10% of non-1,4-polyisoprene by-products (non-target products).
2) When exposed to an acidic environment, 1,4-polyisoprene precipitates and coagulates. Concentrated natural latex is a product obtained by centrifugally concentrating natural latex. It inherits the coagulation property of natural latex upon acid contact, and this characteristic has been enhanced to become an essential attribute in the subsequent production and processing of latex products. Whether used for producing film-based products such as latex gloves, condoms, and balloons, or for manufacturing sponge products like latex mattresses and pillow cores, concentrated latex undergoes a process of transitioning from a fluid latex state to coagulation. This process requires the addition of coagulants, primarily organic acids, and sometimes divalent metal salts. For example, in the production of medical latex products, coagulants for concentrated latex include organic acids such as formic acid, acetic acid, lactic acid, cyclohexylamine acetate, and boric acid. In the Dunlop process for latex mattress production, the coagulant for concentrated latex is hydrofluoric acid generated by the hydrolysis of sodium silicofluoride. In the Talalay process, the coagulant for concentrated latex is carbon dioxide injected after vacuum foaming and freezing, utilizing the acidity of carbonic acid to gradually coagulate and shape the concentrated latex. Synthetic latex is developed to replace natural concentrated latex for the production of various latex products. To meet production requirements, it must possess the same acid-induced coagulation property as natural latex..
3) Natural concentrated latex still contains the rubber protein Hev b 1 allergen.
4) Both concentrated natural latex and synthetic latex contain substantial amounts of substances that are orally toxic to mammals. Whether it is concentrated natural latex or synthetic latex, various orally toxic stabilizers must be added. Synthetic latex additionally contains residual catalysts, solvents, raw material monomers, oligomers, N-nitrosamines and their precursors (potent carcinogens), making it more orally toxic.

Therefore, aside from containing large quantities of orally toxic substances, the most significant issue with both natural concentrated latex and synthetic latex remains their tendency to coagulate immediately upon contact with acid.

In contrast, the 1,4-polyisoprene emulsion dispersion system provided in this application remains dispersed in mammalian gastric fluid, thereby preserving its pharmaceutical activity.

It should be noted that, in the present application, there are no specific limitations regarding the preparation method of the 1,4-polyisoprene emulsion dispersion system. An exemplary preparation method includes, but is not limited to:
Centrifuging natural rubber latex obtained from the Hevea brasiliensis tree at high speed at room temperature, collecting the upper emulsion (removing substances other than rubber particles from the natural rubber latex, including some allergenic protein components), adding an emulsifier, followed by stirring with water to achieve a uniform mixture. After adding alkaline protease isoenzymes (to decompose residual protein allergens within the rubber particles, yielding purified 1,4-polyisoprene), the mixture is incubated and then centrifuged again at high speed. The upper emulsion (emulsifier-coated 1,4-polyisoprene) is collected, diluted with water to a specific mass concentration, and the resulting product is the 1,4-polyisoprene emulsion dispersion system.

In a second aspect, the present application provides a pharmaceutical active ingredient comprising the 1,4-polyisoprene dispersion system according to the first aspect.

In a third aspect, the present application provides a pharmaceutical composition comprising the pharmaceutical active ingredient according to the second aspect.

Preferably, the pharmaceutical composition is used for treating chronic metabolic diseases; Preferably, the chronic metabolic diseases include atherosclerotic cardiovascular and cerebrovascular diseases, type II diabetes, hypercholesterolemia, hypertriglyceridemia, fatty liver disease, colitis, polycystic ovary syndrome (PCOS), and obesity.

Chronic metabolic diseases resulting from long-term nutrient and energy surplus, such as atherosclerotic cardiovascular and cerebrovascular diseases, type II diabetes, hypercholesterolemia, hypertriglyceridemia, obesity, polycystic ovary syndrome, and chronic enteritis, are all classified as chronic inflammatory diseases. Scientific research indicates that nutrient surplus leads to cellular metabolic reprogramming. These metabolites subsequently cause tissue-resident macrophages to polarize toward the pro-inflammatory M1 phenotype, recruiting more inflammatory cells to accumulate in tissues, ultimately resulting in chronic inflammatory lesions in organs and contributing to the onset and progression of diseases. Therefore, developing drugs that target macrophage polarization-inhibiting macrophage foam cell formation, suppressing their shift to the M1 phenotype, and promoting their transition to the M2 phenotype-is an important strategy in the development of treatments for chronic metabolic diseases.

The pharmaceutical active ingredient provided in this application can effectively inhibit macrophage foam cell formation, suppress their polarization to the M1 phenotype, and promote their shift to the M2 phenotype. It can be used for the prevention and treatment of atherosclerotic cardiovascular and cerebrovascular diseases, type II diabetes, hypercholesterolemia, hypertriglyceridemia, fatty liver disease, colitis, polycystic ovary syndrome, and obesity.

In a fourth aspect, the present application provides a use of the pharmaceutical composition in the treatment of Alzheimer's disease, wherein the pharmaceutical composition comprises the pharmaceutical active ingredient according to the second aspect, which in turn comprises the 1,4-polyisoprene dispersion system according to the first aspect.
L-Glutamate, also known as glutamic acid, is an abundant endogenous neurotransmitter in the brains and spinal cords of mammals. It plays important roles in neural development, differentiation, and migration, and is closely associated with synapse formation, development, plasticity, as well as leaming and memory processes. Under normal physiological conditions, glutamate binds to glutamate receptors (GluRs) on the postsynaptic membrane, maintaining normal physiological activity in nerve cells. Under certain pathological stimuli, excessively high extracellular glutamate concentrations lead to excessive calcium ion influx and sustained neuronal depolarization, resulting in overactivation of postsynaptic GluRs. This triggers adverse effects such as excitotoxicity, apoptosis, and oxidative stress, leading to neuronal degeneration and eventual death. Recent studies have shown that many neuropsychiatric disorders, such as Alzheimer's disease (AD), are closely associated with excessively high extracellular glutamate levels.

The pharmaceutical composition provided in this application, which uses the 1,4-polyisoprene dispersion system as the pharmaceutical active ingredient, demonstrates a rescuing effect on L-glutamate (L-GLU)-induced neuronal apoptosis and can be used as a therapeutic agent for Alzheimer's disease

Compared with the prior art, the present application has the following beneficial effects:
(1) The 1,4-polyisoprene dispersion system provided by the present application can be used as a medicinally active ingredient, and it is stable in the gastric acid environment of a mammal, no 1,4-polyisoprene clot is separated out, and there is no oral toxicity to the mammal, and no sensitization source is contained.
(2) Use of the 1,4-polyisoprene dispersion system provided in the present application as a pharmaceutical active ingredient in the manufacture of a medicament for treating chronic metabolic diseases, including atherosclerotic cardiovascular and cerebrovascular diseases, type II diabetes, hypercholesterolemia, hypertriglyceridemia, fatty liver disease, colitis, polycystic ovary syndrome, and obesity. The 1,4-polyisoprene dispersion system significantly inhibits macrophage foam cell formation, suppresses macrophage polarization toward the M1 phenotype, promotes their shift to the M2 phenotype, and concurrently ameliorates inflammatory responses. It demonstrates notable preventive and therapeutic effects against atherosclerotic cardiovascular and cerebrovascular diseases, as well as significant efficacy in treating type II diabetes, hypercholesterolemia, hypertriglyceridemia, fatty liver disease, colitis, obesity, and polycystic ovary syndrome.
(3) The present application demonstrates that both the polyisoprene solution dispersion system and the polyisoprene emulsion dispersion system exhibit a rescuing effect on L-glutamate (L-GLU)-induced neuronal apoptosis. This rescuing effect is dose-dependent, with significant efficacy observed at polyisoprene mass fractions of 20 ppm and 40 ppm (P < 0.05). Both dispersion systems significantly inhibit apoptosis in nerve cells (P < 0.05). Moreover, both the polyisoprene solution dispersion system and the polyisoprene emulsion dispersion system can affect improvement of the cognitive function of AD model mice. Mice treated with the polyisoprene dispersion systems showed significantly shortened escape latency (P < 0.05), along with a significant increase in the number of crossings over the target platform and the target quadrant (P < 0.05). Therefore, the polyisoprene solution dispersion system and the polyisoprene emulsion dispersion system can be used as pharmaceutical active ingredients in the preparation of medicaments for the treatment of Alzheimer's disease.

### DESCRIPTION OF THE DRAWINGS

Figure 1 is an optical micrograph of the 1,4-polyisoprene emulsion dispersion system provided in Example 2.
Figure 2 shows optical micrographs of foamed macrophages from the seven experimental groups in Application Example 1.
Figure 3 is a bar chart showing the test results of foamed macrophage content in total cells from the seven experimental groups in Application Example 1.
Figure 4 shows the test results of M1 macrophage marker gene IL-1β expression levels after LPS treatment in the seven experimental groups in Application Example 2.
Figure 5 shows the test results of M1 macrophage marker gene iNOS expression levels after LPS treatment in the seven experimental groups in Application Example 2.
Figure 6 shows the test results of M2 macrophage marker gene IL-10 mRNA expression levels after LPS treatment in the seven experimental groups in Application Example 2.
Figure 7 shows the test results of M2 macrophage marker gene CD206 expression levels after LPS treatmen in the seven experimental groups t in Application Example 2.
Figure 8 shows the detection results of aortic plaque size in mice from the four experimental groups in Application Example 3.
Figure 9 is a bar chart showing the percentage of aortic plaque area relative to total aortic area in mice from the four experimental groups in Application Example 3.
Figure 10 shows the detection results of aortic root plaque size in mice from the four experimental groups in Application Example 3.
Figure 11 is a bar chart showing aortic root plaque area in mice from the four experimental groups in Application Example 3.
Figure 12 shows the detection results of aortic plaque size in mice from the four experimental groups in Application Example 4.
Figure 13 is a bar chart showing the percentage of aortic plaque area relative to total aortic area in mice from the four experimental groups in Application Example 4.
Figure 14 shows the detection results of aortic root plaque size in mice from the four experimental groups in Application Example 4.
Figure 15 is a bar chart showing aortic root plaque area in mice from the four experimental groups in Application Example 4.
Figure 16 shows the test results of blood glucose levels in mice from the four experimental groups in Application Example 5.
Figure 17 shows the test results of serum glucose levels in mice from the four experimental groups in Application Example 5.
Figure 18 shows the test results of glycated serum protein levels in mice from the four experimental groups in Application Example 5.
Figure 19 shows the test results of HDL cholesterol levels in mice from the four experimental groups in Application Example 6.
Figure 20 shows the test results of LDL cholesterol levels in mice from the four experimental groups in Application Example 6.
Figure 21 shows the test results of total cholesterol levels in mice from the four experimental groups in Application Example 6.
Figure 22 shows the test results of triglyceride levels in mice from the four experimental groups in Application Example 6.
Figure 23 shows the test results of lipoprotein lipase levels in mice from the four experimental groups in Application Example 6.
Figure 24 shows the test results of hepatic lipase levels in mice from the four experimental groups in Application Example 6.
Figure 25 shows the test results of adipose triglyceride lipase levels in mice from the four experimental groups in Application Example 6.
Figure 26 shows the test results of hormone-sensitive lipase levels in mice from the four experimental groups in Application Example 6.
Figure 27 shows photographs of livers from mice in the four experimental groups in Application Example 7.
Figure 28 shows the test results of liver mass as a percentage of total body mass in mice from the four experimental groups in Application Example 7.
Figure 29 shows micrographs of H&E-stained liver sections from mice in the four experimental groups in Application Example 7.
Figure 30 shows micrographs of H&E-stained liver sections from mice in the four experimental groups in Application Example 7.
Figure 31 shows the test results of triglyceride content in liver tissues of mice from the four experimental groups in Application Example 7.
Figure 32 shows the test results of DAI quantitative scores in the four experimental groups in Application Example 8.
Figure 33 shows the test results of weight loss rate in mice from the four experimental groups in Application Example 8.
Figure 34 shows optical photographs of colons collected after euthanasia from mice in the four experimental groups in Application Example 8.
Figure 35 shows the test results of colon length in mice from the four experimental groups in Application Example 8.
Figure 36 shows the test results of colon weight in mice from the four experimental groups in Application Example 8.
Figure 37 shows the test results of body weight in rats from the four experimental groups in Application Example 9.
Figure 38 shows the test results of serum testosterone levels in rats from the four experimental groups in Application Example 9.
Figure 39 shows the test results of fasting blood glucose levels in rats from the four experimental groups in Application Example 9.
Figure 40 shows the test results of blood glucose levels at different time points in rats from the four experimental groups in Application Example 9.
Figure 41 shows the test results of area under the curve (AUC) in oral glucose tolerance tests in rats from the four experimental groups in Application Example 9.
Figure 42 shows the test results of anti-Müllerian hormone (AMH)/GAPDH (mRNA) levels in blood of rats from the four experimental groups in Application Example 9.
Figure 43 shows the test results of IL-1β expression levels in rats from the four experimental groups in Application Example 9.
Figure 44 shows the test results of TNF-α expression levels in rats from the four experimental groups in Application Example 9.
Figure 45 shows the test results of body weight in mice from the four experimental groups in Application Example 10.
Figure 46 shows the test results of fat mass in mice from the four experimental groups in Application Example 10.
Figure 47 shows the test results of adipose tissue morphology in mice from the four experimental groups in Application Example 10.
Figure 48 shows the test results of average adipocyte area in mice from the four experimental groups in Application Example 10.
Figure 49 shows a photograph of gastric contents from mice in the natural latex group in Application Example 11.
Figure 50 shows a photograph of gastric contents from mice in the rubber solution group in Application Example 11.
Figure 51 shows a photograph of gastric contents from mice in the rubber emulsion group in Application Example 11.
Figure 52 shows photographs of the state of 15 groups of 1,4-polyisoprene emulsion dispersion systems under acidic conditions in Application Example 12.
Figure 53 shows the test results of the rescue effect on L-glutamate-induced neuronal apoptosis in 12 experimental groups in a cell model from Application Example 13.
Figure 54 shows the apoptosis detection results by flow cytometry in the four experimental groups in Application Example 14.
Figure 55 is a statistical chart showing the proportion of late apoptotic (Late Apop) cells in the four experimental groups in Application Example 14.
Figure 56 shows the movement trajectories of mice from the four experimental groups in a maze test in Application Example 15.
Figure 57 is a statistical chart of escape latency in mice from the four experimental groups in Application Example 15.
Figure 58 is a statistical chart of the number of crossings over the target platform by mice from the four experimental groups in Application Example 15.
Figure 59 is a statistical chart of the number of crossings through the target quadrant by mice from the four experimental groups in Application Example 15.

### DETAILED DESCRIPTION

The technical solutions of the present application will be further described below in conjunction with the accompanying drawings and specific embodiments. Those skilled in the art should understand that the described embodiments are only intended to aid in the understanding of the present application and should not be construed as specific limitations to the application.

The sources of some components used in the following examples and comparative examples are as described below:
Block-shaped A 1,4-polyisoprene (abbreviated as block 1,4-polyisoprene): Fresh natural rubber latex sourced from Hevea brasiliensis was taken, and 0.1 mol/L hydrochloric acid was added dropwise to coagulate the natural rubber. The coagulated rubber block was then rinsed with water, squeezed to remove moisture, and air-dried to obtain block-shaped 1,4-polyisoprene.

Block-shaped trans-1,4-polyisoprene: Dry eucommia bark and leaves were mechanically crushed and soaked in n-hexane. The extract was concentrated by rotary evaporation, and an excess of 75% ethanol was added to precipitate a gelatinous substance. This substance was redissolved in n-hexane, and 75% ethanol was added again for reprecipitation. The solvent was then evaporated to obtain block-shaped trans-1,4-polyisoprene.

### Example 1

This example provides a 2% by mass cis-1,4-polyisoprene solution dispersion system (hereinafter referred to as rubber solution). The preparation method of the 1,4-polyisoprene solution dispersion system is as follows:
Block-shaped 1,4-polyisoprene was immersed in liquid nitrogen for freezing, then pulverized using a high-speed grinder. The crushed particles were sieved through a 20-mesh screen. 2.0 grams of the sieved particles were weighed and added to 98.0 grams of ethyl oleate. The mixture was stirred continuously at 40°C until the 1,4-polyisoprene particles were completely dissolved, yielding the 1,4-polyisoprene solution dispersion system, i.e., the rubber solution.

### Example 2

This example provides a 10% by mass 1,4-polyisoprene emulsion dispersion system (hereinafter referred to as rubber emulsion). The preparation method of the 1,4-polyisoprene emulsion dispersion system is as follows:
Fresh natural rubber latex from Hevea brasiliensis was centrifuged at high speed at room temperature, and the upper emulsion layer was collected. 1% by weight of the emulsifier polyoxyethylene (20) sorbitan monooleate (purchased from Merck & Co.) was added, followed by the addition of water under stirring to achieve a uniform mixture. Alkaline protease isoenzyme was added to decompose the allergenic protein emulsifiers in the natural latex, and the mixture was incubated at 37°C for 4 hours. It was then centrifuged again at high speed, and the upper emulsion layer was collected. This emulsion was diluted with water to achieve a 1,4-polyisoprene mass percentage of 10% and a polyoxyethylene (20) sorbitan monooleate content of 0.2%, resulting in the 1,4-polyisoprene emulsion dispersion system, i.e., the rubber emulsion.

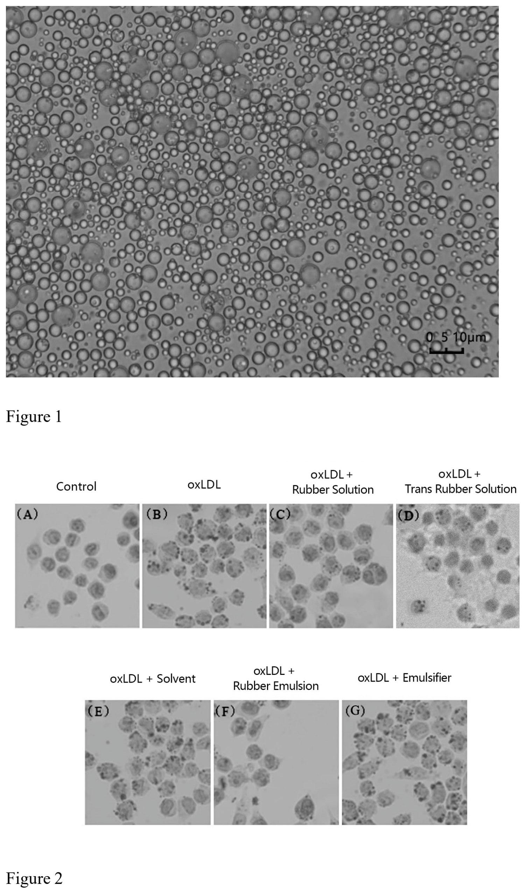

The 1,4-polyisoprene emulsion dispersion system provided in this example was characterized using optical microscopy, and the test results are shown in Figure 1. Figure 1 indicates that the average particle size of the 1,4-polyisoprene emulsion dispersion system provided in this example is ≤10 µm.

### Example 3

This example provides a 2% by mass trans-1,4-polyisoprene solution dispersion system (hereinafter referred to as the trans-rubber solution). Its preparation method is the same as that described in Example 1, with the only difference being that the block-shaped 1,4-polyisoprene is replaced by block-shaped trans-1,4-polyisoprene. All other conditions remain identical to those in Example 1.

### Comparative Example 1

This comparative example provides a rodent diet containing 1,4-polyisoprene particles (hereinafter referred to as rubber particles). The preparation method for this rodent diet containing 1,4-polyisoprene particles is as follows:
Block-shaped 1,4-polyisoprene was immersed in liquid nitrogen until brittle, then pulverized using a high-speed grinder. The crushed particles were sieved through a 20-mesh screen. These particles were added to powdered rodent diet (purchased from Jiangsu Synergy Medical Bioengineering Co., Ltd.) at a ratio of 0.05% (5/10,000) by weight and mixed thoroughly. At this ratio, the daily intake of 1,4-polyisoprene by the mice through the diet is comparable to the amount ingested by the experimental groups via gavage. The mixed feed was then pelletized using a feed pellet machine to produce the mouse diet containing rubber particles, hereafter referred to as "rubber particles."

### Application Example 1: Inhibitory Effect on Macrophage Foam Cell Formation

Oxidized low-density lipoprotein (oxLDL) was used to induce foam cell formation in the RAW264.7 human macrophage cell line. Simultaneously, the macrophages were treated for 24 hours with the rubber solution (dissolved in ethyl oleate, containing 2% by mass of 1,4-polyisoprene), the trans-rubber solution (dissolved in ethyl oleate, containing 2% by mass of trans-1,4-polyisoprene), or the rubber emulsion (containing 10% by mass of 1,4-polyisoprene and 0.2% by mass of the emulsifier polyoxyethylene (20) sorbitan monooleate). The level of macrophage foam cell formation was then assessed using Oil Red O staining.

The experiment was divided into a total of 7 groups, as detailed in Table 1 below:

**Table 1**

| No. | Group Name | Additives and Their Final Concentration in Medium |
|---|---|---|
| 1 | Control Group | / |
| 2 | Oxidized Low-Density Lipoprotein (oxLDL) Group | oxLDL,40µg/mL |
| 3 | oxLDL + Rubber Solution Group | oxLDL,40µg/mL+Rubber Solution, 100 µg/mL |
| 4 | oxLDL + Trans-Rubber Solution Group | oxLDL,40µg/mL+ Trans-Rubber Solution, 100 µg/mL |
| 5 | oxLDL + Solvent Group | oxLDL, 40µg/mL + Ethyl Oleate, 98µg/mL |
| 6 | oxLDL + Rubber Emulsion Group | oxLDL, 40µg/mL + Rubber Emulsion, 100µg/mL |
| 7 | oxLDL + Emulsifier Group | oxLDL,40µg/mL+Emulsifier, 0.2µg/mL |

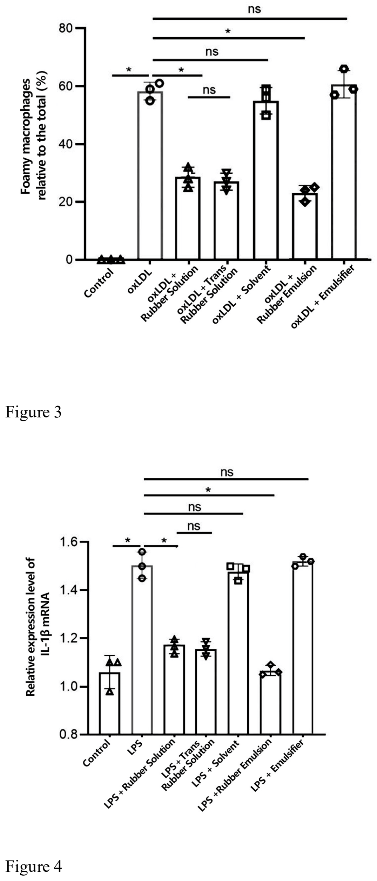

Using an optical microscope, the morphology of macrophages from the aforementioned seven experimental groups was examined. The test results are shown in Figure 2 (Figure 2(A) shows macrophages from the control group, Figure 2(B) shows macrophages from the oxLDL group, Figure 2(C) shows macrophages from the oxLDL + rubber solution group, Figure 2(D) shows macrophages from the oxLDL + trans-rubber solution group, Figure 2(E) shows macrophages from the oxLDL + solvent group, Figure 2(F) shows macrophages from the oxLDL + rubber emulsion group, and Figure 2(G) shows macrophages from the oxLDL + emulsifier group). Additionally, the percentage of foamy macrophages relative to the total cell count in these seven groups was statistically analyzed. The histogram of foamy macrophage content is shown in Figure 3. It should be noted that in Figure 3, "ns" indicates no statistically significant difference, while an asterisk (*) indicates a statistically significant difference (the same applies below).

As shown in Figures 2-3, compared to the oxLDL group, both the rubber solution group (including cis and trans structures) and the rubber emulsion group significantly inhibited macrophage foam formation, resulting in a lower degree of foamification. In contrast, the solvent group and the emulsifier group showed no significant effect on macrophage foam formation, resulting in a relatively high degree of foam formation.

Furthermore, Figures 2-3 indicate that the cis-trans structure of 1,4-polyisoprene has no significant impact on drug activity.

### Application Example 2: Effect on Macrophage Polarization

The human macrophage cell line RAW264.7 was treated for 24 hours with the rubber solution provided in Example 1, the rubber emulsion provided in Example 2, or the trans-rubber solution provided in Example 3. Subsequently, the cells were induced to polarize for 10 hours using lipopolysaccharide (LPS). The expression of marker genes associated with macrophage polarization was detected via real-time quantitative PCR.

The experiment was divided into a total of 7 groups, as detailed in Table 2 below:

**Table 2**

| No. | Control Group | Additives and Their Final Concentration in Medium |
|---|---|---|
| 1 | Control Group | / |
| 2 | Lipopolysaccharide (LPS) Group | LPS,1 µg/mL |
| 3 | LPS+Rubber Solution Group | LPS,I µg/mL+Rubber Solution, 100µg/mL |
| 4 | LPS+Trans-Rubber Solution Group | LPS,I µg/mL+Trans-Rubber Solution, 100 µg/mL |
| 5 | LPS+Solvent Group | LPS,I µg/mL+Ethyl Oleate, 98µg/mL |
| 6 | LPS+Rubber Emulsion Group | LPS,1 µg/mL+Rubber Emulsion, 100 µg/mL |
| 7 | LPS+Emulsifier Group | LPS,I µg/mL+Emulsifier, 0.2µg/mL |

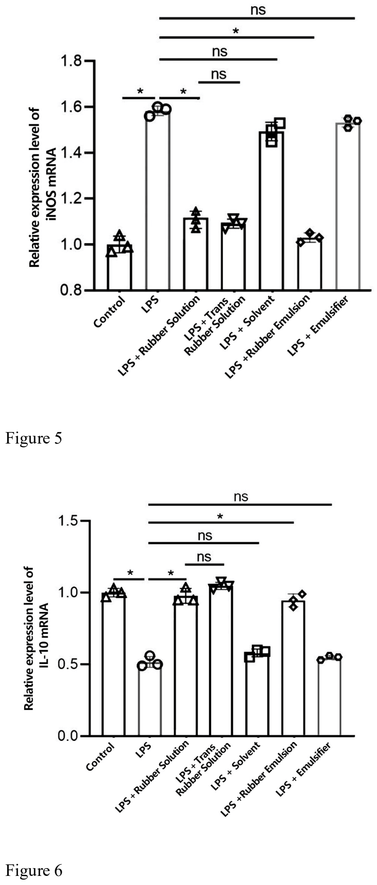

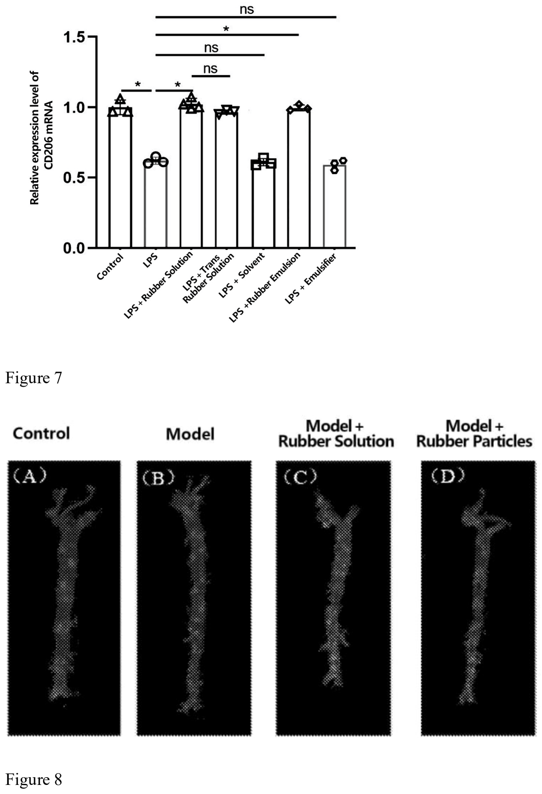

Using a real-time quantitative PCR instrument, the mRNA content of the aforementioned seven experimental groups was detected. The expression levels of M1 macrophage marker genes IL-1β and iNOS after LPS treatment are shown in Figures 4 and 5, respectively, while the expression levels of M2 macrophage marker genes CD206 and IL-10 mRNA after LPS treatment are shown in Figures 6 and 7, respectively.

As shown in Figures 4-7, after LPS treatment, the expression of M1 macrophage marker genes IL-1β and iNOS was upregulated, while the expression of M2 phenotype marker genes IL-10 and CD206 was downregulated. Compared to the LPS group, rubber solution and rubber emulsion significantly reduced the mRNA expression levels of IL-1β and iNOS and increased the mRNA expression levels of CD206 and IL-10. Both the rubber solution group and the trans-rubber solution group exhibited activity without statistically significant differences. This indicates that both rubber solution and rubber emulsion, including cis and trans structures, can inhibit macrophage polarization toward the M1 phenotype and promote polarization toward the M2 phenotype, while also ameliorating inflammatory responses. However, the solvent and emulsifier had no significant effect on macrophage polarization.

Additionally, as shown in Figures 4-7, the cis-trans structure of 1,4-polyisoprene had no significant impact on drug activity.

### Application Example 3: Preventive Study on Atherosclerosis

This application example is a preventive study, meaning the drug intervention began before the experimental mice developed atherosclerotic plaques, to investigate the preventive effects of the drug on atherosclerosis.

Twenty-eight 8-week-old male apoE-/- mice were randomly divided into four groups of seven mice each: control group, atherosclerosis model group (referred to as model group), model + rubber solution group, and model + rubber particles group.

The control group was fed a normal diet for 16 weeks, while the other three groups were fed a high-fat diet (21% fat, 0.5% cholesterol) for 16 weeks. The atherosclerosis model group, model + rubber solution group, and model + rubber particles group were administered ethyl oleate, rubber solution, and ethyl oleate, respectively, via oral gavage daily at a dose of 3 g/kg body weight. Additionally, the feed for the model + rubber particles group was supplemented with rubber particles. The grouping of the experimental mice is shown in Table 3 below:

**Table 3**

| No. | Group Name | Diet Condition |
|---|---|---|
| 1 | Control Group | Normal Diet |
| 2 | Atherosclerosis Model Group (Model Group) | High-Fat Diet+Ethyl Oleate |
| 3 | Model + Rubber Solution Group | High-Fat Diet+Rubber Solution |
| 4 | Model + Rubber Particles Group | High-Fat Diet+Ethyl Oleate+Rubber Particles |

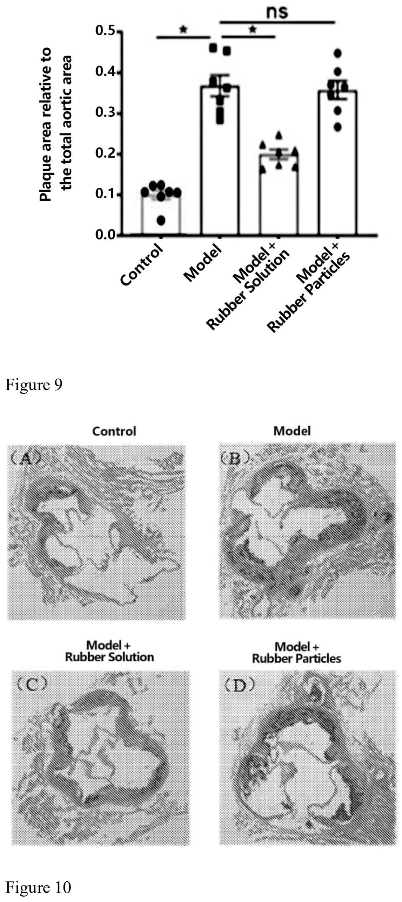

After the experiment, the mouse aorta and root were collected. The aortic root was stained with Oil Red O, and the positive areas were quantified using ImageJ software to measure the size of atherosclerotic plaques in the four experimental groups. The entire aorta was also stained with Oil Red O, and the results of plaque size detection are shown in Figure 8 (Figure 8(A) shows the control group, Figure 8(B) shows the atherosclerosis model group, Figure 8(C) shows the model + rubber solution group, and Figure 8(D) shows the model + rubber particles group). The percentage of positive area (plaque area) relative to the total aortic area is shown in Figure 9.

As shown in Figures 8-9, the rubber solution significantly inhibited plaque progression in atherosclerotic mice, while the rubber particles group showed no significant change in plaque content.

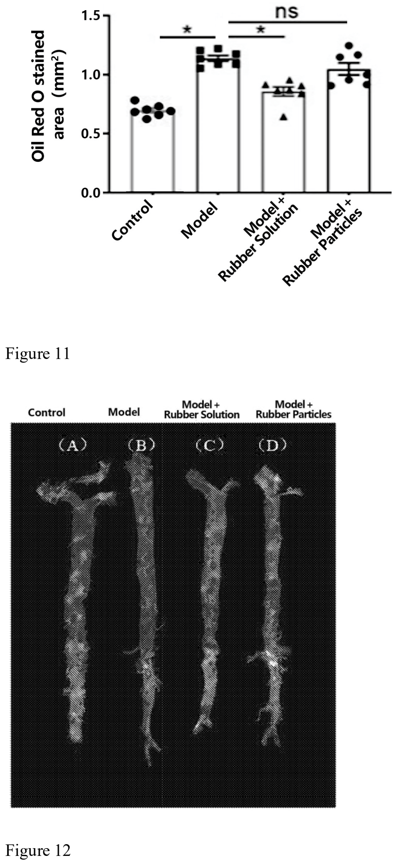

The entire aortic root was stained with Oil Red O, and the plaque size detection results are shown in Figure 10 (Figure 10(A) shows the control group, Figure 10(B) shows the atherosclerosis model group, Figure 10(C) shows the model + rubber solution group, and Figure 10(D) shows the model + rubber particles group). The bar graph of the positive area (plaque area) is shown in Figure 11.

As shown in Figures 10-11, the rubber solution significantly inhibited the increase of plaques in atherosclerotic mice, while the group fed with rubber particles showed no significant in plaque content.

The occurrence and progression of atherosclerotic plaques are key to cardiovascular and cerebrovascular diseases. This experiment confirms that the rubber solution has a preventive effect in mice that have not yet developed atherosclerotic plaques, slowing the progression of atherosclerosis and reducing plaque volume. Rubber particles did not demonstrate any preventive effect.

### Application Example 4: Therapeutic Study on Atherosclerosis

This application example is a therapeutic study, where drug intervention began after atherosclerotic plaques had already formed in the experimental mice, to investigate the therapeutic effects of the drug on atherosclerosis.

Twenty-eight 8-week-old male apoE-/- mice were randomly divided into four groups of seven mice each: control group, atherosclerosis model group, model + rubber solution group, and model + rubber particles group.

The control group was fed a normal diet for 16 weeks, while the other three groups were fed a high-fat diet (21% fat, 0.5% cholesterol) for 16 weeks. Starting from week 12, the atherosclerosis model group, model + rubber solution group, and model + rubber particles group were administered ethyl oleate, rubber solution, and ethyl oleate, respectively, via oral gavage daily at a dose of 3 g/kg body weight. Additionally, the high-fat diet of the model + rubber particles group was supplemented with rubber particles from week 12 onward. The grouping of the experimental mice is shown in Table 4 below.

**Table 4**

| No. | Group Name | Diet Condition |
|---|---|---|
| 1 | Control Group | Normal Diet |
| 2 | Atherosclerosis Model Group (Model Group) | High-Fat Diet+ add Ethyl Oleate after 12 weeks |
| 3 | Model + Rubber Solution Group | High-Fat Diet+add Rubber Solution after 12 weeks |
| 4 | Model + Rubber Particles Group | High-Fat Diet+add Ethyl Oleate and Rubber Particles after 12 weeks |

After the experiment, the mouse aorta and its root were collected. The aortic root was stained with Oil Red O, and the positive areas were quantified using ImageJ software to measure the size of atherosclerotic plaques in the aforementioned four experimental groups.

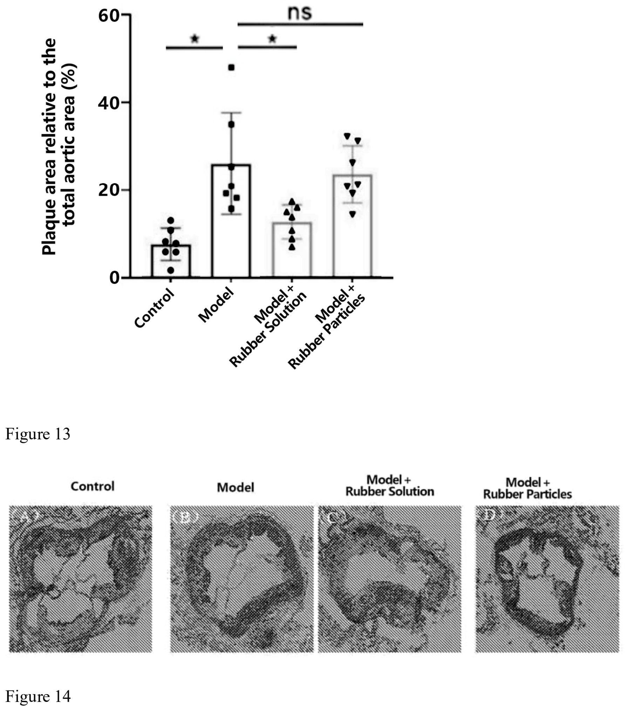

The entire aorta was stained with Oil Red O, and the results of plaque size detection are shown in Figure 12 (Figure 12(A) shows the control group, Figure 12(B) shows the atherosclerosis model group, Figure 12(C) shows the model + rubber solution group, and Figure 12(D) shows the model + rubber particles group). The percentage of po sitive area (plaque area) relative to the total aortic area is shown in Figure 13.

As shown in Figures 12-13, the rubber solution significantly inhibited plaque progression in atherosclerotic mice, while the rubber particles group showed no significant change in plaque content.

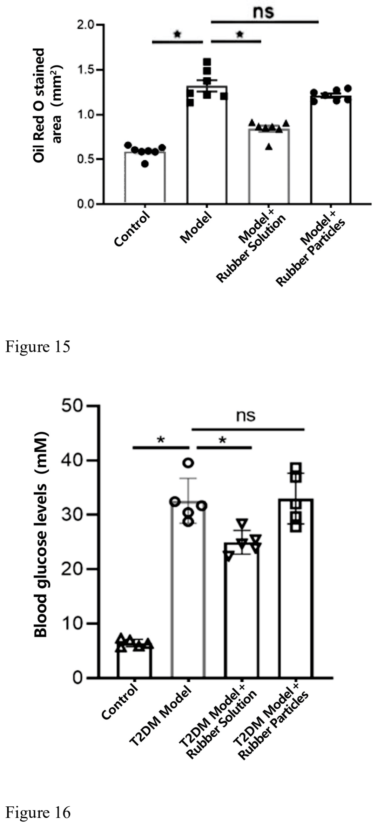

The entire aortic root was stained with Oil Red O, and the plaque size detection results are shown in Figure 14 (Figure 14(A) shows the control group, Figure 14(B) shows the atherosclerosis model group, Figure 14(C) shows the model + rubber solution group, and Figure 14(D) shows the model + rubber particles group). The bar graph of the positive area (plaque area) is shown in Figure 15.

As shown in Figures 14-15, the rubber solution significantly suppressed plaque formation in atherosclerotic mice, while the rubber particles group exhibited no significant.

The development and progression of atherosclerotic plaques are critical in cardiovascular and cerebrovascular diseases. This experiment confirms that the rubber solution has a therapeutic effect in mice that have already developed atherosclerotic plaques, slowing the progression of atherosclerosis and reducing plaque volume. Rubber particles did not demonstrate any therapeutic effect.

### Application Example 5: Therapeutic Study on type II diabetes

Five 7-week-old SPF male m/m mice were used as the control group. Fifteen 7-week-old SPF male db/db mice were randomly divided into three groups of five mice each: type II diabetes group, type II diabetes + rubber solution group, and type II diabetes + rubber particles group. Random blood glucose levels were measured on two different days, and if the blood glucose level was ≥16.7 mmol/L on both days, the model was considered successfully established.

After successful modeling, the type II diabetes + rubber solution group was administered rubber solution via oral gavage once daily at a dose of 3 g/kg body weight for 12 consecutive weeks. The type II diabetes + rubber particles group was administered ethyl oleate via oral gavage once daily at a dose of 3 g/kg body weight, and their feed was replaced with feed containing rubber particles for 12 consecutive weeks.

The following tests were performed on the control group, type II diabetes group, type II diabetes + rubber solution group, and type II diabetes + rubber particles group:
(1) Fasting blood glucose (FBG) measurement: Mice were fasted for over 12 hours with free access to water. The tail was disinfected with alcohol, and blood was collected from the tail vein. The second drop of blood was used for blood glucose detection using a Roche glucose meter.
(2) Serum glucose (GLU) and glycated serum protein (GSP) content measurement: Whole blood was collected to prepare serum, and an automatic biochemical analyzer was used to detect serum glucose and glycated serum protein levels.

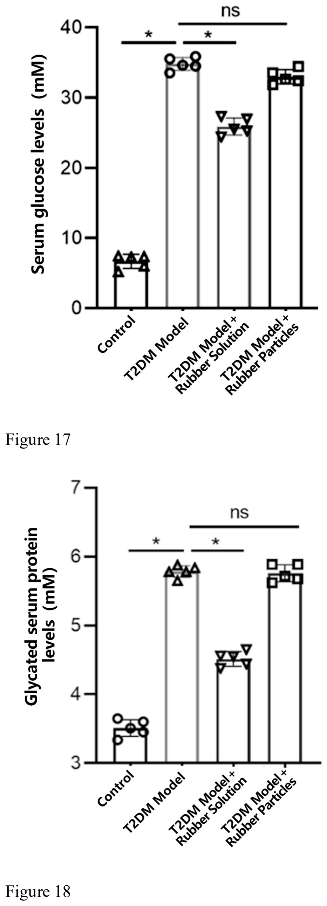

The test results are shown in Figures 16-18. As shown in Figures 16-18, the rubber solution reduced blood glucose, serum glucose, and glycated serum protein levels in diabetic model mice, demonstrating a therapeutic effect on diabetes. In contrast, rubber particles showed no therapeutic effect on diabetes.

### Application Example 6: Therapeutic Study on Hypertriglyceridemia and Hypercholesterolemia

Twenty 7-week-old apoE-/- mice were equally divided into four groups of five mice each: control group, model group, model + rubber solution group, and model + rubber particles group. The total experimental period was 8 weeks, with the first 4 weeks dedicated to establishing a hyperlipidemia mouse model and the subsequent 4 weeks for drug treatment.

Throughout the 8-week feeding period, the control group was fed a standard diet, while the other three groups were maintained on a high-sugar high-fat diet (Product No.: D12108C, purchased from Research Diets, Inc.).

During weeks 5-8, the control group, model group, model + rubber solution group, and model + rubber particles group were administered pure water, ethyl oleate, rubber solution, and ethyl oleate, respectively, via oral gavage once daily at a dose of 3 g/kg body weight for 4 consecutive weeks. Additionally, during weeks 5-8, the feed for the model + rubber particles group was replaced with feed containing rubber particles.

An automatic biochemical analyzer was used to measure the serum levels of total cholesterol (TC), triglycerides (TG), high-density lipoprotein cholesterol (HDL-C), and low-density lipoprotein cholesterol (LDL-C) in each group.

Enzyme-linked immunosorbent assay (ELISA) was employed to determine the levels of triglyceride-related hydrolases in the serum of each group, including: adipose triglyceride lipase (ATGL), lipoprotein lipase (LPL), hepatic lipase (HL), and hormone-sensitive triglyceride lipase (HSL).

Adipose triglyceride lipase (ATGL) and hormone-sensitive triglyceride lipase (HSL) are the primary lipolytic enzymes in mammals, accounting for over 95% of triglyceride hydrolase activity in white adipose tissue. The catabolism of triglycerides (TG) primarily occurs through the action of ATGL and HSL, which are key rate-limiting enzymes in lipolysis. Hepatic lipase (HL) is mainly synthesized and secreted by liver parenchymal cells and participates in lipid metabolism.

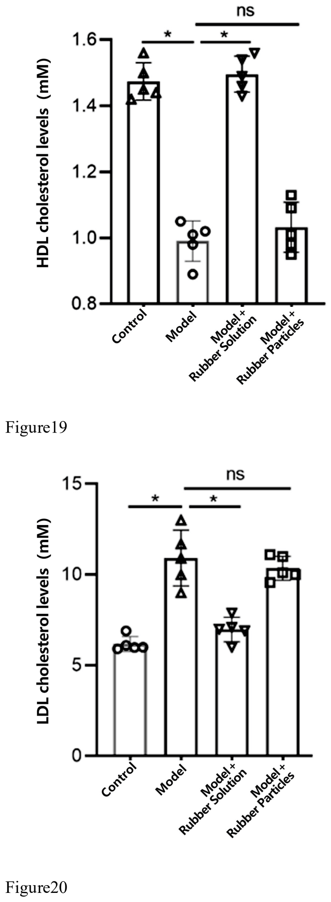

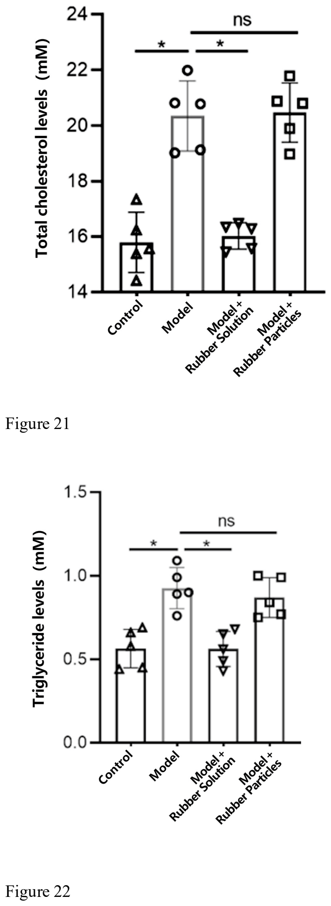

As shown in Figures 19-22, the rubber solution reduced the levels of total cholesterol, triglycerides, and LDL-C in the blood lipids while increasing HDL-C levels. In contrast, rubber particles had no significant effect on blood lipids. This indicates that the rubber solution has a significant therapeutic effect on hypercholesterolemia and hypertriglyceridemia, while rubber particles show no therapeutic effect on these conditions.

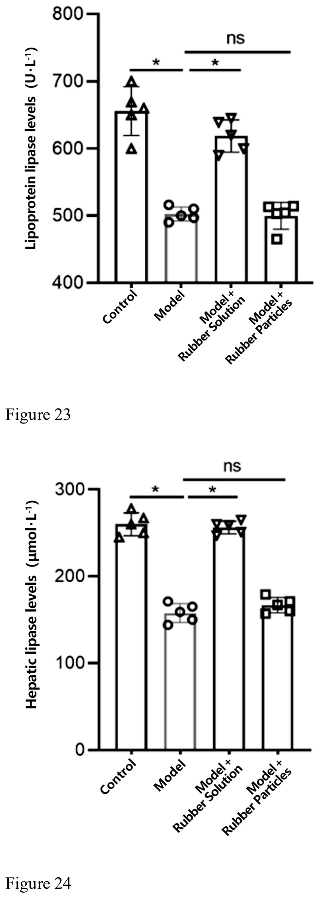

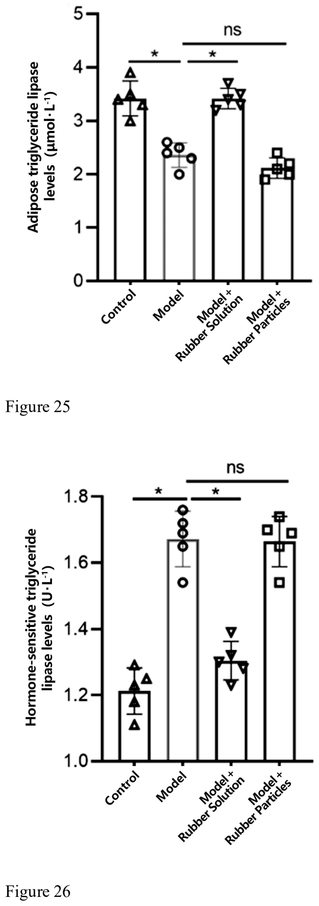

As shown in Figures 23-26, the rubber solution significantly increased the levels of triglyceride metabolism-related enzymes-ATGL, LPL, and HL-while significantly decreasing HSL levels. Rubber particles had no significant effect on these enzymes. This demonstrates that the rubber solution promotes TG hydrolysis, indicating a significant therapeutic effect on hypertriglyceridemia, whereas rubber particles show no therapeutic effect.

### Application Example 7: Therapeutic Study on Fatty Liver

Twenty 6-week-old male C57BL/6 mice were randomly divided into four groups of five mice each: control group, fatty liver model group (referred to as model group), model + rubber solution group, and model + rubber particles group.

The control group was fed a standard diet for 14 weeks, while the other three groups were fed a high-fat diet (21% fat, 0.15% cholesterol) for 14 weeks. The feed for the model + rubber particles group was additionally supplemented with rubber particles. Simultaneously, the control group, fatty liver model group, model + rubber solution group, and model + rubber particles group were administered pure water, ethyl oleate, rubber solution, and ethyl oleate, respectively, via oral gavage once daily at a dose of 3 g/kg body weight for 14 weeks.

After the experiment, the livers and tissues of the mice were collected for photography and weighing.

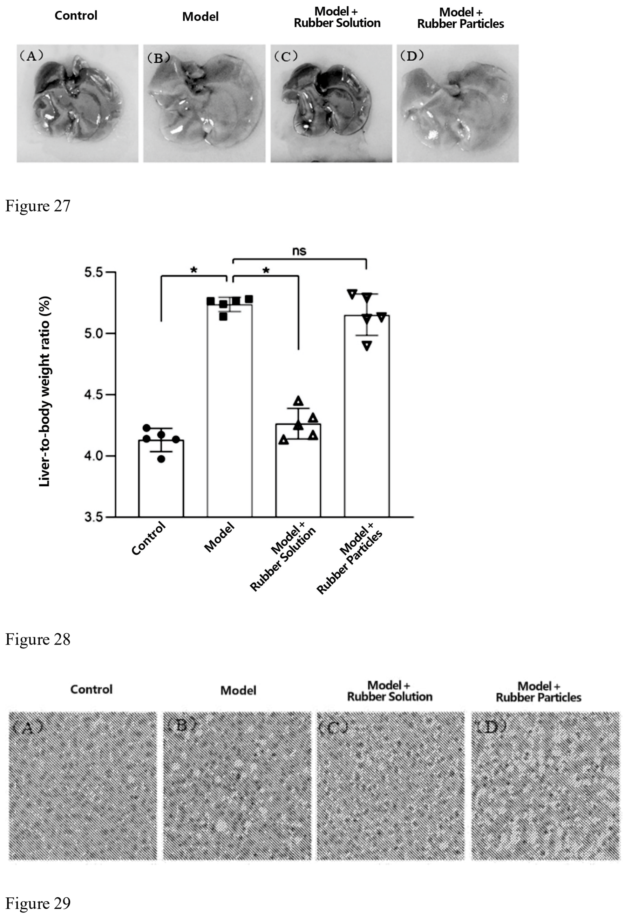

Morphological photographs of the livers from each group and the statistical graph of liver weight as a percentage of total body weight are shown in Figures 27-28 (Figure 27(A): control group; Figure 27(B): model group; Figure 27(C): model + rubber solution group; Figure 27(D): model + rubber particles group). Figure 27 shows that compared to the control group, the model group successfully developed fatty liver; liver fat accumulation was significantly inhibited in the rubber solution group; and the rubber particles group showed no significant change compared to the model group. Figure 28 shows that the model group had significantly increased liver weight compared to the control group, while the rubber solution group significantly reversed the weight gain associated with fatty liver caused by lipid accumulation. The rubber particles group showed no significant change compared to the model group.

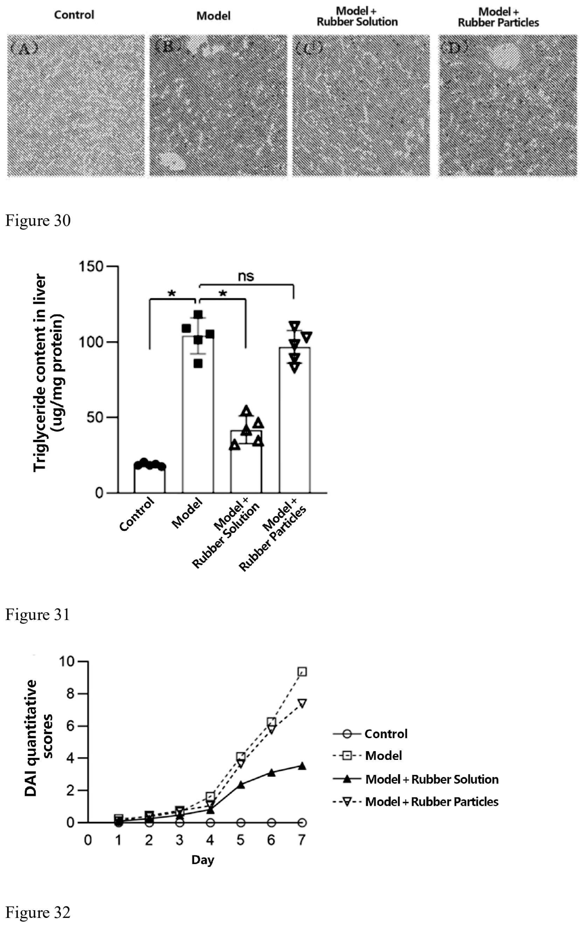

Further, liver tissue sections from each group were prepared and stained with H&E and Oil Red O. The stained liver sections were examined under an optical microscope, and the results are shown in Figures 29-30. Figure 29(A) and Figure 30(A) show H&E and Oil Red O staining of the control group, respectively; Figure 29(B) and Figure 30(B) show the model group; Figure 29(C) and Figure 30(C) show the model + rubber solution group; and Figure 29(D) and Figure 30(D) show the model + rubber particles group.

Figure 29 shows the morphology of hepatocytes via H&E staining. Compared to the control group, the fatty liver model group exhibited extensive necrotic cells forming necrotic cores. In contrast, the rubber solution group showed intact hepatocyte structure, tight arrangement, minimal necrosis, and no necrotic cores, indicating significant reversal of fatty liver lesions. The rubber particles group had numerous necrotic cells forming necrotic cores, similar to the model group.

Figure 30 shows fat distribution in hepatocytes via Oil Red O staining. Compared to the model group, the rubber solution significantly inhibited the increase in fat induced by the high-fat diet in fatty liver mice, while the rubber particles group showed no significant change in fat content.

The triglyceride (TG) content in liver tissue is a quantitative indicator of fatty liver severity. A tissue triglyceride (TG) enzymatic assay kit was used to measure TG levels in the liver tissue of each group, and the results are shown in Figure 31. Figure 31 shows that compared to the model group, oral administration of rubber solution significantly reduced liver TG levels, while the rubber particles group showed no significant change.

The above results demonstrate that the rubber solution significantly inhibits the progression of high-fat diet-induced fatty liver and has therapeutic effects on fatty liver, while rubber particles lack this efficacy.

### Application Example 8: Therapeutic Study on Colitis

Thirty-two 8-week-old male mice were randomly divided into four groups of eight mice each: control group, colitis model group (referred to as model group), model + rubber solution group, and model + rubber particles group.

The experiment lasted 7 days. The control group was fed a standard diet, while the other three groups were fed a diet supplemented with 4% dextran sodium sulfate (DSS) to induce colitis symptoms. The feed for the model + rubber particles group was additionally supplemented with rubber particles. Simultaneously, the control group, colitis model group, model + rubber solution group, and model + rubber particles group were administered pure water, ethyl oleate, rubber solution, and ethyl oleate, respectively, via oral gavage once daily at a dose of 3 g/kg body weight for 7 days.

Body weight was measured and recorded daily during the experiment. Additionally, feces were collected daily to observe stool consistency and the presence of occult blood or bloody stools. These data were used to calculate the Disease Activity Index (DAI) score.

The DAI (Disease Activity Index) is a comprehensive score based on the percentage of weight loss, stool consistency, and stool bleeding. The DAI scoring criteria are shown in Table 5.

**Table 5**

| Weight Loss Rate (%) | Stool Character* | Fecal Occult Blood/Gross Bloody Stool | Score |
|---|---|---|---|
| 0 | normal | normal | 0 |
| 1-5 | loose | Occult Blood Positive | 1 |
| 5-10 | Loose | Occult Blood Positive | 2 |
| 10-15 | Watery Stool | Gross Bloody Stool | 3 |
| >15 | Watery Stool | Gross Bloody Stool | 4 |

In the table above, normal stool refers to formed stool; loose stool refers to paste-like, semi-formed stool that does not adhere to the anus; and watery stool refers to thin, watery stool that can adhere to the anus.

The DAI score quantifies the severity of colitis lesions in mice. A higher DAI score indicates more severe colitis lesions. The quantitative DAI scoring results are shown in Figure 32. As seen in Figure 32, compared to the colitis model group, the model + rubber solution group had a lower DAI score, while the model + rubber particles group showed no significant difference from the colitis model group. This indicates that the rubber solution alleviates colitis symptoms in mice, whereas rubber particles do not have this effect.

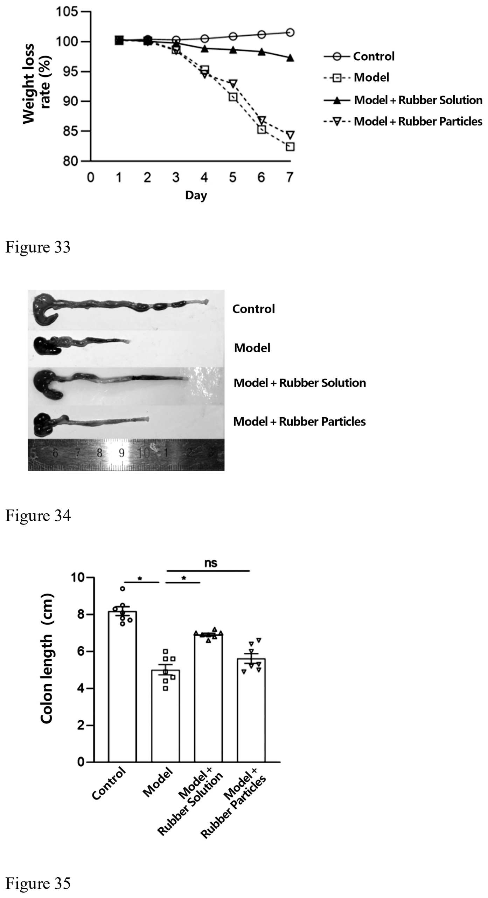

Under otherwise identical conditions, weight loss in mice is also an indicator of colitis severity. The statistical graph of weight loss rates in the four experimental groups is shown in Figure 33. As seen in Figure 33, the weight of mice in the colitis model group gradually decreased over the experimental period; weight loss in the model + rubber solution group was slower compared to the colitis model group; and the model + rubber particles group showed no significant difference in weight loss compared to the colitis model group. This further confirms that the rubber solution alleviates colitis symptoms in mice, while rubber particles do not.

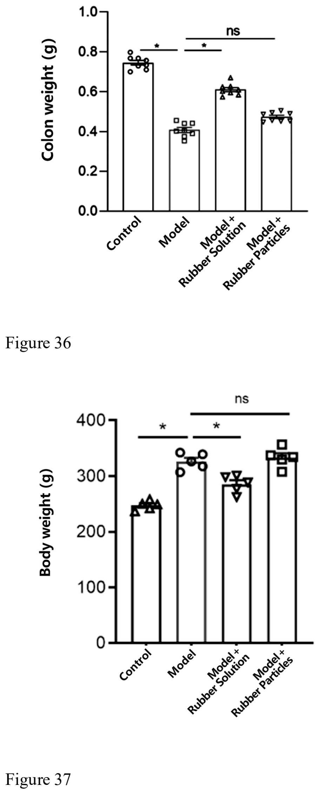

The length and weight of the mouse colon are also quantitative indicators of colitis severity. More severe colitis results in a shorter and lighter colon. After euthanizing the mice on day 7, the colons were collected and photographed, as shown in Figure 34. The colon length and weight of each group were measured, and the results are shown in Figures 35 and 36, respectively. As seen in Figures 34-36, compared to the control group, the colitis model group had shorter and lighter colons. However, the model + rubber solution group showed less severe colon changes compared to the colitis model group, while the model + rubber particles group showed no significant difference in colon length or weight compared to the colitis model group. This demonstrates that the rubber solution has a therapeutic effect on colitis in mice, whereas rubber particles do not.

### Application Example 9: Therapeutic Study on Polycystic Ovary Syndrome (PCOS)

Twenty 4-week-old female SD rats were randomly divided into four groups of five rats each: control group, PCOS model group (referred to as model group), model + rubber solution group, and model + rubber particles group.

The experiment lasted 3 weeks. During this period, except for the control group, the other three groups were treated with letrozole via oral gavage once daily for 3 weeks to establish the PCOS model. Simultaneously, the control group, PCOS model group, model + rubber solution group, and model + rubber particles group were administered pure water, ethyl oleate, rubber solution, and ethyl oleate, respectively, via oral gavage once daily at a dose of 3 g/kg body weight for 3 weeks. The feed for the model + rubber particles group was replaced with feed containing rubber particles.

After the gavage treatment, rat body weight was measured, and blood was collected from the tail vein to prepare serum for testosterone level measurement. After fasting for 12 hours, blood was collected from the tail vein to measure fasting blood glucose using a glucose meter. Then, glucose (10 g/100 mL) was intraperitoneally injected at a dose of 2 g/kg, and blood was collected from the tail vein at 15, 30, 60, 90, and 120 minutes after injection to measure blood glucose levels. The rats were euthanized, and ovarian tissues were collected to extract RNA. The mRNA expression levels of anti-Müllerian hormone (AMH), IL-1β, and TNF-α were detected using real-time quantitative PCR.

As the PCOS model was established, rat body weight gradually increased. The extent of weight gain reflects the severity of PCOS. The body weights of the four groups are shown in Figure 37. As seen in Figure 37, rubber solution gavage significantly reduced PCOS-induced weight gain, while rubber particles had no significant effect on body weight.

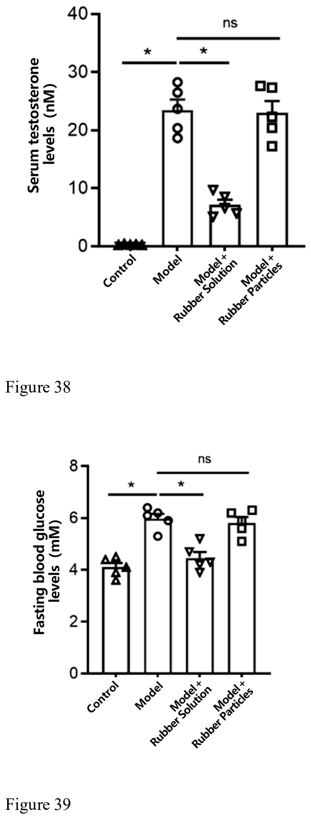

A key feature of PCOS is elevated androgen levels. Testosterone levels can indicate the severity of PCOS. The serum testosterone levels of the four groups are shown in Figure 38. Rubber solution gavage significantly suppressed the increase in serum testosterone in PCOS model rats, while rubber particles had no significant effect compared to the model group.

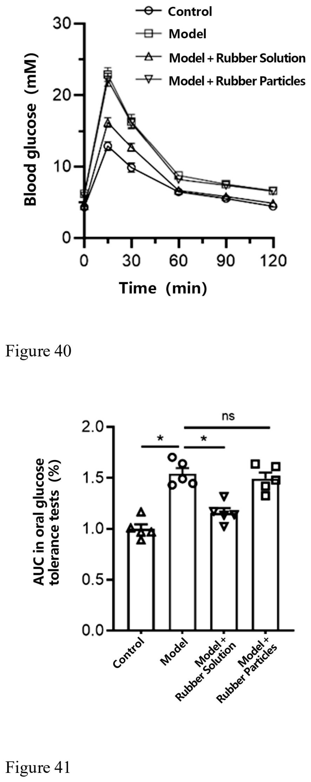

Impaired glucose tolerance is a common metabolic abnormality in PCOS. The degree of impairment reflects the severity of PCOS. Fasting blood glucose, blood glucose levels at different time points, and glucose tolerance were tested in the four groups, and the results are shown in Figures 39-41. As seen in Figures 39-41, the PCOS model group showed increased fasting blood glucose and impaired glucose tolerance. However, rubber solution gavage significantly improved these abnormalities, while rubber particles had no significant effect.

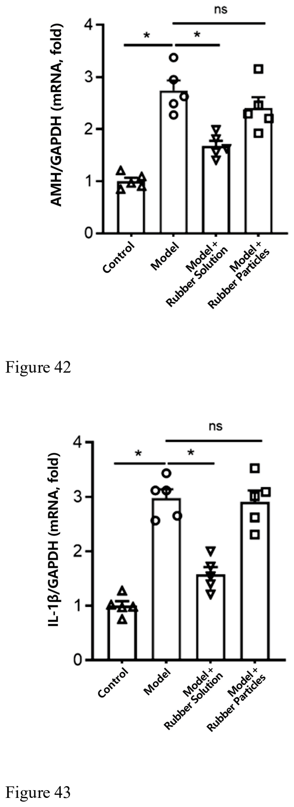

Elevated anti-Müllerian hormone (AMH) levels are a marker of follicular development disorder in PCOS patients. The AMH/GAPDH (mRNA) levels (normalized to the GAPDH reference gene) in the blood of the four groups are shown in Figure 42. As seen in Figure 42, AMH mRNA levels were significantly elevated in the ovarian tissue of the PCOS model group. Rubber solution gavage, but not rubber particles, reduced AMH mRNA levels.

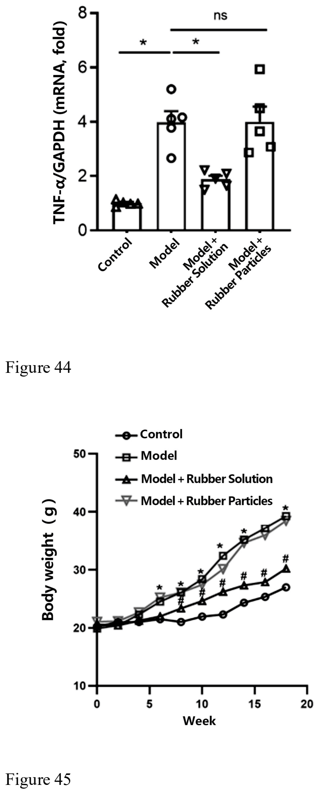

Ovarian tissue in PCOS patients exhibits persistent chronic inflammation. Increased expression of chronic inflammatory factors IL-1β and TNF-α can serve as indicators of disease severity. The expression levels of IL-1β and TNF-α in the blood of the four groups are shown in Figures 43 and 44, respectively. As seen in Figures 43-44, compared to the control group, the PCOS model group showed significantly increased expression of IL-1β and TNF-α in ovarian tissue. Rubber solution gavage significantly downregulated the expression of these inflammatory factors, while rubber particles had no significant effect.

In summary, comprehensive comparison of multiple indicators in PCOS rats-including body weight, serum testosterone levels, fasting blood glucose, glucose tolerance, ovarian AMH levels, and inflammatory factor expression-demonstrates that rubber solution has therapeutic efficacy for PCOS, while rubber particles do not.

### Application Example 10: Therapeutic Study on Obesity

Twenty-four 6-week-old male C57BL/6 mice were randomly divided into four groups of six mice each: control group, high-fat model group, high-fat + rubber solution group, and high-fat + rubber particles group. The average body weight of the mice was 20 ± 2 g.

The experiment lasted 18 weeks. The control group was fed a standard diet, while the other three groups were fed a high-fat diet (35% fat content). The feed for the high-fat + rubber particles group was supplemented with rubber particles. The control group, high-fat model group, high-fat + rubber solution group, and high-fat + rubber particles group were administered pure water, flower ethyl oleate, rubber solution, and ethyl oleate, respectively, via oral gavage once daily at a dose of 3 g/kg body weight for 18 weeks.

Body weight was recorded weekly, and fat mass was analyzed using a body composition analyzer at the end of the experiment. The mice were euthanized, and epididymal adipose tissue was collected for sectioning and H&E staining to calculate the average adipocyte area.

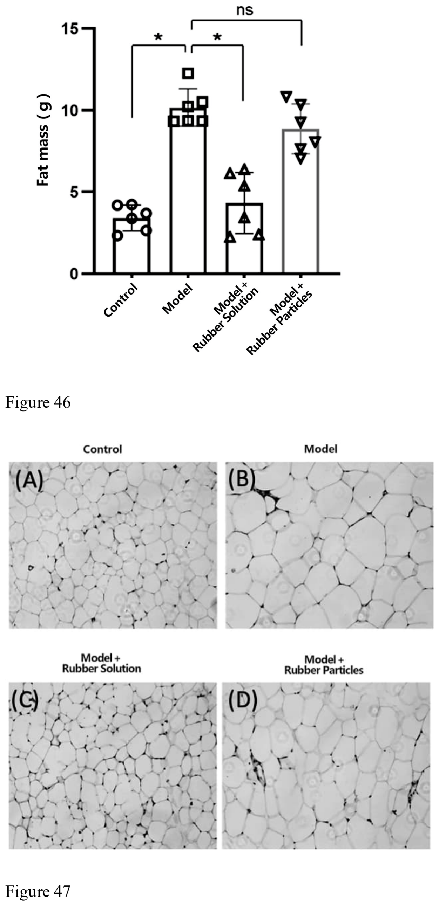

The body weight and fat mass results for the four groups are shown in Figures 45 and 46, respectively. As seen in Figure 45, compared to the control group, the high-fat model group showed significant weight gain. Rubber solution gavage significantly inhibited high-fat diet-induced weight gain, while rubber particles had no inhibitory effect. As seen in Figure 46, fat mass data were consistent with body weight data: rubber solution significantly inhibited high-fat-induced increases in fat mass, while rubber particles had no effect.

Adipose tissue morphology was examined under an optical microscope, and the results are shown in Figure 47 (Figure 47(A): control group; Figure 47(B): high-fat model group; Figure 47(C): model + rubber solution group; Figure 47(D): model + rubber particles group). As seen in Figure 47, adipocytes in the control group were small and neatly arranged; adipocytes in the high-fat group were enlarged with some inflammatory cell infiltration; rubber solution significantly inhibited high-fat diet-induced adipocyte enlargement; and the cell morphology in the rubber particles group was similar to that of the high-fat group.

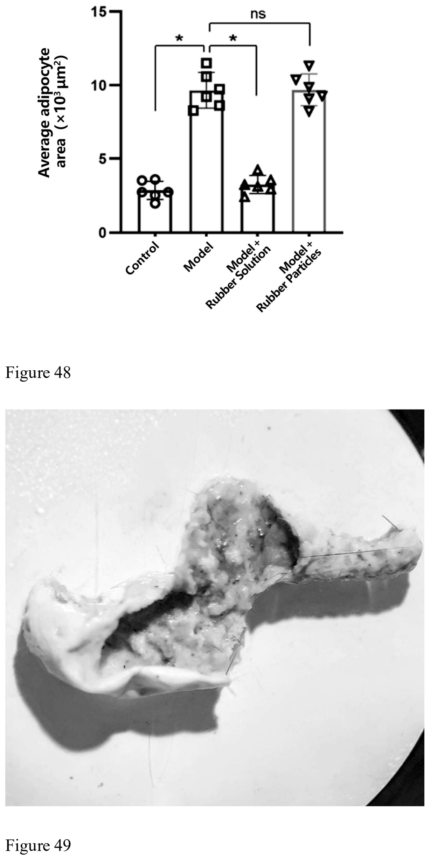

The average adipocyte area was measured under an optical microscope, and the results are shown in Figure 48. As seen in Figure 48, statistical analysis of adipocyte area revealed that the average adipocyte area in the high-fat group was 2.8 times that of the control group; rubber solution significantly inhibited adipocyte area expansion, with an average adipocyte area only 1.2 times that of the control group; and the rubber particles group showed no significant change compared to the high-fat group.

In summary, comprehensive comparison of data including body weight, fat mass, adipose tissue histology, and average adipocyte area demonstrates that rubber solution significantly inhibits high-fat diet-induced obesity and has therapeutic efficacy for obesity, while rubber particles do not.

### Application Example 11 Study on the State of Natural Rubber Latex, Rubber Emulsion, and Rubber Solution in the Mouse Stomach

Fifteen 6-week-old male C57BL/6 mice were randomly divided into three groups of five mice each: natural latex group, rubber emulsion group, and rubber solution group. The experiment lasted 7 days, with all groups fed a standard diet.

The natural latex group, rubber emulsion group, and rubber solution group were administered natural latex from Hevea brasiliensis, the rubber emulsion provided in this application, and the rubber solution provided in this application, respectively, via oral gavage once daily at a dose of 3 g/kg body weight for 7 days. Feces were collected daily and examined with a dissecting microscope. On the last day, the dose was increased to 15 g/kg body weight, and the mice were euthanized within 30 minutes after gavage. Gastric contents were collected and examined with a dissecting microscope.

The gastric contents of the natural latex group, rubber solution group, and rubber emulsion group were examined, and the results are shown in Figures 49-51. As seen in Figures 49-51, rubber coagulum was observed in the gastric contents of the natural latex group, while no rubber coagulum was observed in the rubber emulsion or rubber solution groups.

Thus, natural rubber latex coagulates into large rubber lumps in the mouse stomach, failing to maintain a dispersed state and lacking pharmacological activity, before being excreted through the digestive tract. In contrast, the rubber emulsion and rubber solution provided in this application do not coagulate into large lumps, remain dispersed, and exhibit pharmacological activity.

### Application Example 12 Stability Study of Rubber Emulsions Prepared with Different Emulsifiers in Strong and Weak Acidic Environments

To evaluate the stability of natural latex from Hevea brasiliensis, industrial concentrated latex (including high-ammonia, low-ammonia, and low-protein concentrated latex) prepared from natural latex, and synthetic 1,4-polyisoprene latex under acidic conditions-and to compare them with the 1,4-polyisoprene emulsion system prepared with the emulsifiers preferred in this application-the inventors simulated mammalian gastric acid environments in vitro using HCl solutions at pH=1 and pH=3. The former represents the strong acidity of gastric juice, while the latter represents weak acidity.

The method for preparing the 1,4-polyisoprene emulsion system with the emulsifiers preferred in this application can refer to the method provided in Example 2, with the only difference being the emulsifier used.

Stability under weak acid (pH=3): Add 5 mL of pH=3 HCl solution (1.0 mmol/L) to a petri dish, pipette 0.1 mL of 1,4-polyisoprene emulsion into it, mix well, and observe whether solids precipitate. If solids precipitate, the emulsion system is unstable under weak acid conditions; otherwise, it is stable.

Stability under strong acid (pH=1): Add 5 mL of pH=1 HCl solution (0.1 mol/L) to a petri dish, pipette 0.1 mL of 1,4-polyisoprene emulsion into it, mix well, and observe whether solids precipitate. If solids precipitate, the emulsion system is unstable under strong acid conditions; otherwise, it is stable.

The descriptions and test results of different 1,4-polyisoprene emulsion dispersion systems are detailed in Table 6 below:

**Table 6**

| Code | 1,4-Polyisoprene Emulsion System | Source and Description | Is it stable under weak acid conditions at pH=3 | Is it stable under strong acid conditions at pH=1 |
|---|---|---|---|---|
| A | Natural Latex from Hevea brasiliensis | Collected from Hevea brasiliensis | Unstable | Unstable |
| B | Industrial High-Ammonia Concentrated Latex, 0.6% | Hainan Rubber Group HSF Concentrated Natural Rubber Latex (CNR) | Unstable | Unstable |
| C | Industrial Low-Ammonia Concentrated Latex, Ammonia 0.2% | Yunnan Xishuangbanna Yuncheng Rubber Co., Ltd. TZ Low-Ammonia Concentrated Latex | Unstable | Unstable |
| D | Industrial Low-Protein Concentrated Latex, Nitrogen 0.2% | Yunnan Xishuangbanna Yuncheng Rubber Co., Ltd. TZ-Free Deproteinized Latex | Unstable | Unstable |
| E | Synthetic Isoprene Latex | Kraton Corporation, USA, , kraton IR401 Latex | Unstable | Unstable |

| | | | | |
|---|---|---|---|---|
| F | Emulsion System Prepared with Polyoxyethylene (20) Sorbitan Monolaurate (Tween-20) as Emulsifier | Emulsifier from Merck Company | Stable | Stable |
| G | Emulsion System Prepared with Polyoxyethylene (20) Monopalmitate (Tween-40) as Emulsifier | Emulsifier from Merck Company | Stable | Stable |
| H | Emulsion System Prepared with Polyoxyethylene (20) Monostearate (Tween-60) as Emulsifier | Emulsifier from Merck Company | Stable | Stable |
| I | Emulsion System Prepared with Polyoxyethylene (20) Sorbitan Monooleate (Tween-80) | Emulsifier from Merck Company | Stable | Stable |
| J | Emulsion System Prepared with Decaglycerol Monolaurate (Q-12S) as Emulsifier | Emulsifier from Jinan Dongrun Fine Chemicals Technology Co., Ltd | Stable | Stable |
| K | Emulsion System Prepared with Decaglycerol Monomyristate (Q-14S) as Emulsifier | Emulsifier from Jinan Dongrun Fine Chemicals Technology Co., Ltd | Stable | Stable |
| L | Emulsion System Prepared with Decaglycerol Monopalmitate (Q-16S) as Emulsifier | Emulsifier from Jinan Dongrun Fine Chemicals Technology Co., Ltd | Stable | Stable |
| M | Emulsion System Prepared with Decaglycerol Monooleate (Q-17S) as Emulsifier | Emulsifier from Jinan Dongrun Fine Chemicals Technology Co., Ltd | Stable | Stable |
| N | Emulsion System Prepared with Decaglycerol Monostearate (Q-18S) as Emulsifier | Emulsifier from Jinan Dongrun Fine Chemicals Technology Co., Ltd | Stable | Stable |
| O | Emulsion System Prepared with Sucrose Monostearate as Emulsifier | mulsifier from Shanghai Macklin Biochemical Technology Co., Ltd | Stable | Stable |

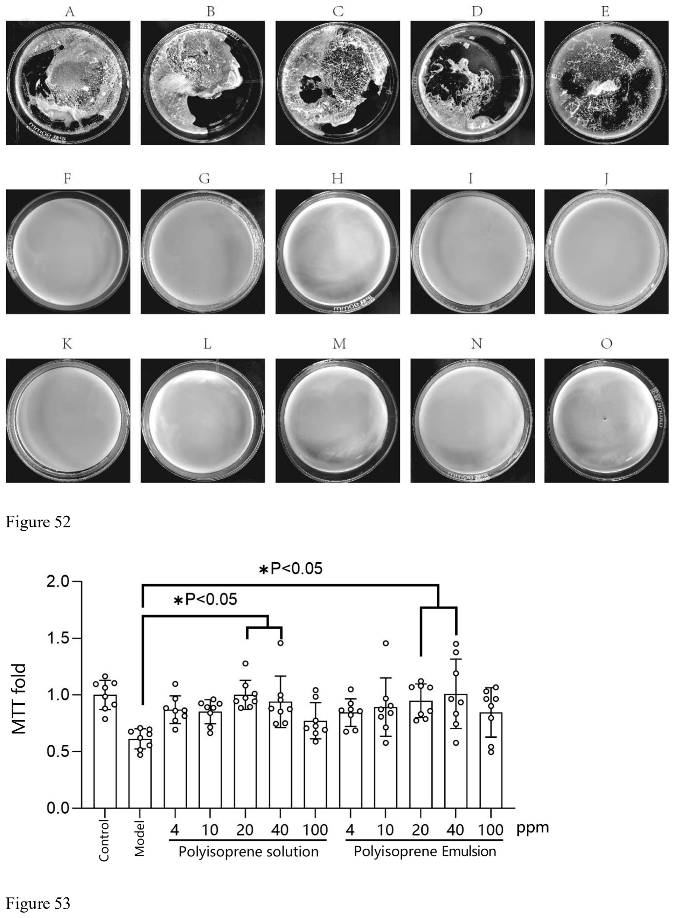

Figure 52 shows partial test results, wherein:
A is a photograph of natural latex from *Hevea brasiliensis* dropped into hydrochloric acid at pH=3;
B is a photograph of high-ammonia concentrated natural latex dropped into hydrochloric acid at pH=3;
C is a photograph of low-ammonia concentrated natural latex dropped into hydrochloric acid at pH=3;
D is a photograph of low-protein concentrated natural latex dropped into hydrochloric acid at pH=3;
E is a photograph of synthetic isoprene latex dropped into hydrochloric acid at pH=3;
F is a photograph of the rubber emulsion prepared in this application using polyoxyethylene (20) sorbitan monolaurate (Tween-20) as the emulsifier, dropped into hydrochloric acid at pH=1;
G is a photograph of the rubber emulsion prepared in this application using polyoxyethylene (20) sorbitan monopalmitate (Tween-40) as the emulsifier, dropped into hydrochloric acid at pH=1;
H is a photograph of the rubber emulsion prepared in this application using polyoxyethylene (20) sorbitan monostearate (Tween-60) as the emulsifier, dropped into hydrochloric acid at pH=1;
I is a photograph of the rubber emulsion prepared in Example 2 of this application using polyoxyethylene (20) sorbitan monooleate (Tween-80) as the emulsifier, dropped into hydrochloric acid at pH=1;
J is a photograph of the rubber emulsion prepared in this application using decaglycerol monolaurate (Q-12S) as the emulsifier, dropped into hydrochloric acid at pH=1;
K is a photograph of the rubber emulsion prepared in this application using decaglycerol myristate (Q-14S) as the emulsifier, dropped into hydrochloric acid at pH=1;
L is a photograph of the rubber emulsion prepared in this application using decaglycerol palmitate (Q-16S) as the emulsifier, dropped into hydrochloric acid at pH=1;
M is the result of the rubber emulsion prepared in this application using decaglycerol monooleate (Q-17S) as the emulsifier, dropped into hydrochloric acid at pH=1;
N is a photograph of the rubber emulsion prepared in this application using decaglycerol stearate (Q-18S) as the emulsifier, dropped into hydrochloric acid at pH=1;
O is a photograph of the rubber emulsion prepared in this application using sucrose monostearate as the emulsifier, dropped into hydrochloric acid at pH=1.

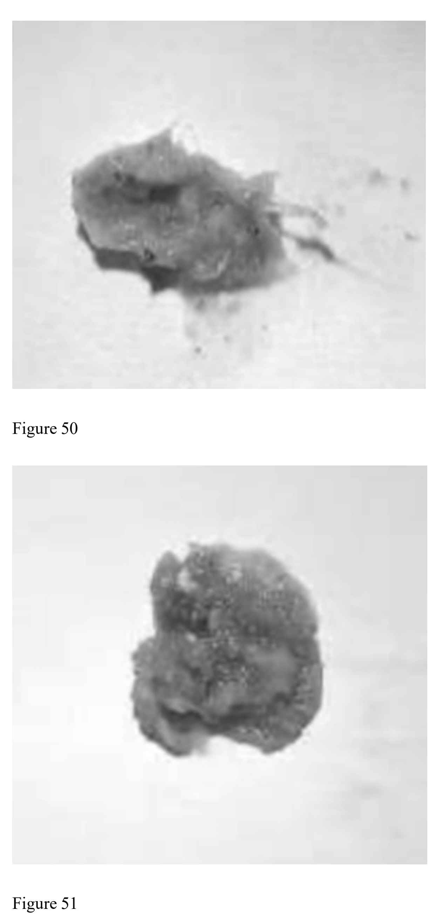

As can be seen from Table 6 and Figure 50, the natural latex from *Hevea brasiliensis* in the prior art, industrial concentrated latex prepared from natural latex as raw material (including high-ammonia concentrated latex, low-ammonia concentrated latex, and low-protein concentrated latex), as well as synthetic 1,4-polyisoprene latex, are all unstable under acidic conditions at pH=1 and pH=3, causing the 1,4-polyisoprene to coagulate and precipitate. This indicates that it would also coagulate and precipitate in mammalian gastric fluid, unable to maintain a dispersed state, and will not produce pharmacological activity.

The emulsion systems preferably prepared in this application using polyoxyethylene (20) sorbitan monolaurate (Tween-20), polyoxyethylene (20) sorbitan monopalmitate (Tween-40), polyoxyethylene (20) sorbitan monostearate (Tween-60), polyoxyethylene (20) sorbitan monooleate (Tween-80), decaglycerol monolaurate (Q-12S), decaglycerol myristate (Q-14S), decaglycerol palmitate (Q-16S), decaglycerol monooleate (Q-17S), decaglycerol stearate (Q-18S), and sucrose monostearate as emulsifiers, all remained stable under acidic conditions at pH=1.0 and pH=3.0. This indicates that the rubber emulsion prepared therefrom will also maintain a dispersed state in mammalian gastric fluid, thereby exerting pharmacological activity.

### Application Example 13: Rescue of MTT Cell Viability in a Neuronal Apoptosis Model by the Polyisoprene Dispersion System

### Introduction to the L-Glutamate-Induced Neuronal Apoptosis Model:

L-Glutamate is an abundant endogenous neurotransmitter in the brain and spinal cord of mammals. It plays an important role in nerve development, differentiation, and migration, and is also closely related to synaptogenesis, development, plasticity, and learning and memory processes. Under normal physiological conditions, glutamate binds to glutamate receptors (GluR) on the postsynaptic membrane, maintaining normal physiological activities of nerve cells. Under certain pathological stimuli, excessively high extracellular glutamate concentration leads to excessive calcium ion influx and sustained depolarization of neurons, thereby overactivating GluR on the postsynaptic membrane. This subsequently triggers adverse reactions such as excitotoxicity, apoptosis, and oxidative stress, leading to neuronal degeneration and eventual death. Recent research indicates that many neuropsychiatric diseases, such as Alzheimer's disease (AD), are closely related to excessively high extracellular glutamate concentration. Therefore, the glutamate-induced neuronal apoptosis model is widely used in disease research and drug screening for Alzheimer's disease (AD).

In this application example, the neuronal cells are the HT-22 cell line, derived from mouse hippocampal neurons. By detecting the MTT cell viability indicator, the rescue effects of different concentrations of polyisoprene dispersion systems (rubber solution system, rubber emulsion system) on apoptotic HT-22 cells were compared. MTT is 3-(4,5-dimethylthiazol-2-yl)-2,5-diphenyltetrazolium bromide, and the MTT assay is a method for detecting cell survival and growth.

### Grouping:

12 groups were set up, namely: 1. Control group; 2. L-GLU model group; 3-7. Rubber solution groups (polyisoprene mass fractions of 4 ppm, 10 ppm, 20 ppm, 40 ppm, and 100 ppm respectively; the polyisoprene in these 5 groups all came from the 2% mass percentage 1,4-polyisoprene solution dispersion system prepared in Example 1); 8-12. Polyisoprene emulsion treatment groups (polyisoprene mass fractions of 4 ppm, 10 ppm, 20 ppm, 40 ppm, and 100 ppm respectively; the polyisoprene in these 5 groups all came from the 10% mass percentage 1,4-polyisoprene emulsion dispersion system prepared in Example 2, which was then diluted 5-fold with pure water to obtain a 2% 1,4-polyisoprene emulsion dispersion system).

The additives for these 12 groups of cells and their final concentrations in the culture medium are shown in Table 7 below:

**Table 7**

| No. | Group Name | Additives and Their Final Concentration in Medium |
|---|---|---|
| 1 | Contrast | / |
| 2 | L-GLU Modeling Group | L-GLU 20 mM |
| 3 | Rubber Solution Group 4ppm | 20 mM+4µg/mL Polyisoprene (add 0.2µL of 2% Rubber Solution to 1 mL Cell Medium) |
| 4 | Rubber Solution Group10ppm | L-GLU 20 mM+10 µg/mLPolyisoprene (add 0.5µL of 2% Rubber Solution to 1mL Cell Medium) |
| 5 | Rubber Solution Group 20ppm | L-GLU 20 mM+20µg/mLPolyisoprene (add 1µL of 2% Rubber Solution to 1 mL Cell Medium) |
| 6 | Rubber Solution Group 40ppm | L-GLU 20 mM+40µg/mLPolyisoprene (add 2µL of 2% Rubber Solution to 1 mL Cell Medium) |
| 7 | Rubber Solution Group 100ppm | L-GLU 20 mM+100µg/mLPolyisoprene (add 5µL of 2% Rubber Solution to 1 mL Cell Medium) |
| 8 | Rubber Emulsion Group 4ppm | L-GLU 20 mM+4µg/mLPolyisoprene (add 0.2µL of 2% Rubber Emulsion to 1 mL Cell Medium) |
| 9 | Rubber Emulsion Group 10ppm | L-GLU 20 mM+10µg/mLPolyisoprene (add 0.5µL of 2% Rubber Emulsion to 1 mL Cell Medium) |
| 10 | Rubber Emulsion Group 20ppm | L-GLU 20 mM+20µg/mLPolyisoprene (add 1µL of 2% Rubber Emulsion to 1 mL Cell Medium) |
| 11 | Rubber Emulsion Group 40ppm | L-GLU 20 mM + 40µg/mL Polyisoprene (add 2µL of 2% Rubber Emulsion to 1 mL Cell Medium) |
| 12 | Rubber Emulsion Group 100ppm | L-GLU 20 mM + 100µg/mL Polyisoprene (add 5µL of 2% Rubber Emulsion to 1 mL Cell Medium) |

### Experimental Procedure:

(1) Culture: A cell suspension was prepared using a culture medium containing 10% fetal bovine serum. Cells were seeded at 5,000 cells per well in a 96-well plate, with a cell suspension volume of 200 µL per well.
(2) According to the grouping described above, corresponding substances were added to the 12 groups of cells, with 8 replicate wells set for each group. The cells were incubated for 24 hours.
(3) After the culture period, 20 µL of MTT solution (5 mg/mL, prepared in serum-free and antibiotic-free medium) was added to each well. After 4 hours of incubation, the culture was terminated. The medium in the wells was carefully aspirated and discarded, and 150 µL of DMSO was added to each well. The plate was shaken on a rocker at room temperature for 15 minutes to fully dissolve the crystals.
(4) The optical absorbance of each well was measured at a wavelength of 550 nm using an enzymelinked immunosorbent assay (ELISA) reader, and the results were recorded.

### Result Analysis:

The experimental results are shown in Figure 53. The results indicate that polyisoprene in either a solution dispersion system or an emulsion dispersion system has a rescuing effect on L-glutamate (L-GLU)-induced neuronal apoptosis, and this rescuing effect is dose-dependent. Specifically, polyisoprene at mass fractions of 20 ppm and 40 ppm showed significant rescuing effects (P < 0.05). However, at mass fractions below 20 ppm or above 40 ppm, the rescuing effects were not significant (P > 0.05).

### Application Example 14: Polyisoprene Dispersion System Reduces the Proportion of Late Apoptotic Cells in a Neuronal Apoptosis Model

### Introduction to the L-Glutamate-Induced Neuronal Apoptosis Model:

L-Glutamate (L-Glutamate), also known as glutamate, is an abundant endogenous neurotransmitter in the brains and spinal cords of mammals. It plays a crucial role in neuronal development, differentiation, and migration, and is closely associated with synaptogenesis, synaptic development, plasticity, and learning and memory processes. Under normal physiological conditions, glutamate binds to glutamate receptors (GluRs) on the postsynaptic membrane, maintaining normal physiological activities of nerve cells. Under certain pathological stimuli, excessive extracellular glutamate concentrations lead to excessive calcium ion influx, sustained depolarization of neurons, and overactivation of GluRs on the postsynaptic membrane. This, in turn, triggers adverse effects such as excitotoxicity, apoptosis, and oxidative stress, causing neuronal degeneration and eventual death. Recent studies have shown that many neuropsychiatric disorders, such as Alzheimer's disease (AD), are closely related to excessively high extracellular glutamate concentrations. Therefore, the glutamate-induced neuronal apoptosis model is widely used in disease research and drug screening for Alzheimer's disease (AD).

In this application example, the neuronal cells used were the HT-22 cell line, derived from mouse hippocampal neurons. The rescuing effect of the polyisoprene dispersion system (rubber solution system and rubber emulsion system) on apoptotic HT-22 cells was evaluated by detecting the proportion of HT-22 cells in the late apoptotic stage (Late Apop).

### Grouping:

In this application example, a total of four groups of HT-22 mouse hippocampal neuronal cells were set up, with three replicate wells for each group.
- Group 1: Control group, where cells were cultured with medium only without any additives.
- Group 2: L-GLU model group, where 20 mM L-glutamate was added to induce neuronal apoptosis.
- Group 3: Rubber solution group, where 20 mM L-glutamate and polyisoprene at a mass fraction of 20 ppm (20 µg/mL) were added. The polyisoprene was derived from the 2% 1,4-polyisoprene solution dispersion system prepared in Example 1.
- Group 4: Rubber emulsion group, where 20 mM L-glutamate and polyisoprene at a mass fraction of 20 ppm (20 µg/mL) were added. The polyisoprene was derived from the 10% 1,4-polyisoprene emulsion dispersion system prepared in Example 2, which was diluted 5-fold with pure water to obtain a 2% 1,4-polyisoprene emulsion dispersion system.

The additives for these four groups of cells and their final concentrations in the culture medium are shown in Table 8 below:

**Table 8**

| No. | Group Name | Additives and Their Final Concentration in Culture Medium |
|---|---|---|
| 1 | Control Group | / |
| 2 | L-GLU Modeling Group | L-GLU 20mM |
| 3 | Rubber Solution Group | L-GLU 20mM+20µg/mLPolyisoprene (add 1µL of 2% Rubber Solution to 1 mL Cell Medium) |
| 4 | Rubber Emulsion Group | L-GLU 20mM+20µg/mLPolyisoprene (add 1µL of 2% Rubber Emusion to 1 mL Cell Medium) |

### Specific Experimental Procedure:

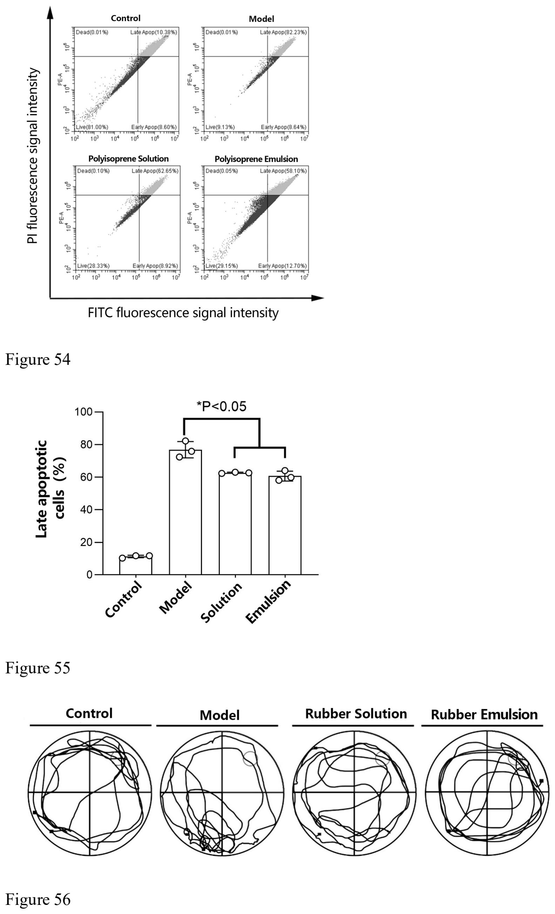

(1) HT-22 cells were seeded into 6-well plates, with 3 replicate wells per group. After the cells adhered, they were co-treated for 24 hours with the polyisoprene solution dispersion system or the polyisoprene emulsion dispersion system along with 20 mM L-Glu. The mass fraction of polyisoprene in the cell culture medium was 20 ppm (20 µg/mL) for both treatment groups. The control group received no additions. The L-GLU model group was treated only with 20 mM L-Glutamate for 24 hours.
(2) After treatment, the cells were digested using trypsin (without EDTA) and then washed twice with pre-chilled PBS.
(3) After washing, the cells were incubated with Annexin V-PE and PI working solution in the dark at room temperature for 20 minutes. Apoptosis was then detected using a flow cytometer. Each of the 3 replicate wells per group was detected once. Typical detection results are shown in Figure 54.
(4) The proportion of cells in the late apoptotic stage (Late Apop) for each group was statistically analyzed. The results are shown in Figure 55.

### Result Analysis:

In Figure 54, the cells in the upper right quadrant (marked by the crosshairs), represented by light gray dots, are in the late apoptotic stage (Late Apop). The corresponding numbers indicate the proportion of late apoptotic cells.

From Figure 55, it can be observed that the proportion of late apoptotic cells significantly increased in the model group after treatment with L-Glutamate. However, after treatment with 20 µg/mL polyisoprene, the proportion of late apoptotic cells significantly decreased, demonstrating that polyisoprene has a significant inhibitory effect on apoptosis in the nerve cells of this model (P < 0.05). Polyisoprene, whether from the solution dispersion system or the emulsion dispersion system, had the same effect, with no difference between them.

### Application Example 15: Animal In Vivo Experiment, Mouse Morris Water Maze Model

### Animal Model, Grouping, and Experimental Method:

This application example used male APP/PS1 mice on a C57BL/6J background; these are double transgenic AD model mice. The animal room temperature was maintained at 24 ± 2°C with a 12h light/dark cycle. Food intake, water consumption, and body weight were checked daily during the experiment. Male APP/PS1 mice on a C57BL/6J background (2 months old, weighing about 20g) and their wild-type (WT) littermate control mice were purchased from GemPharmatech Co., Ltd. (Nanjing, Jiangsu, China). All animals had free access to food and drinking water.

C57BL/6J wild-type mice fed a standard diet served as the normal control group (WT group, n=12 per group).

36 APP/PS1 mice were randomly divided into the following 3 groups (n=12 per group) and received the following treatments:
AD Model Group: APP/PS1 mice fed a standard diet.

Rubber Solution Group: APP/PS1 mice given a standard diet and orally gavaged daily with 100 µL of the 2% mass percentage 1,4-polyisoprene solution dispersion system prepared in Example 1.

Rubber Emulsion Group: APP/PS1 mice given a standard diet and orally gavaged daily with 100 µL of a 2% 1,4-polyisoprene emulsion dispersion system. This 2% emulsion was prepared by diluting the 10% mass percentage 1,4-polyisoprene emulsion dispersion system from Example 2 five-fold with pure water.

All mice underwent 7 months of feeding and treatment. After the treatment period, a water maze test was conducted. The movement trajectories of the mice were recorded to visually observe changes in spatial memory across the groups.

### Result Analysis:

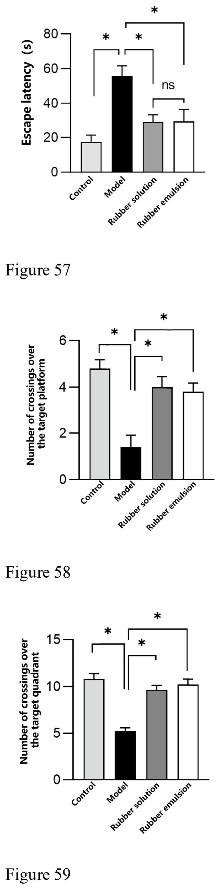

The test results of the water maze model for the mice in the above 4 experimental groups are shown in Figures 56-59. Figure 56 records the movement trajectories of mice in each group, with the topright quadrant being the target quadrant. Figure 57 is a statistical graph of the escape latency for each group. Figure 58 is a statistical graph of the number of times mice in each group crossed the target platform location. Figure 59 is a statistical graph of the number of times mice in each group crossed the target quadrant.

From Figures 56-59, it can be seen that compared to mice in the AD model group, mice treated with the polyisoprene dispersion system showed a significantly shortened escape latency (P < 0.05), and significantly increased numbers of crossings over the target platform location and the target quadrant (P < 0.05).

This means that polyisoprene significantly improved the cognitive function of AD model mice and thus has the potential to be developed into a drug for treating AD. Polyisoprene, whether in a solution dispersion state or an emulsion dispersion state, exhibited therapeutic activity for AD.

### Summary:

From the above content, it is understood that in this application, by dispersing 1,4-polyisoprene in either a solution dispersion system or an emulsion dispersion system, it remains stable in the mammalian gastric acid environment without the precipitation of 1,4-polyisoprene clots, is orally non-toxic to mammals, and contains no allergens. Dispersed 1,4-polyisoprene can significantly inhibit macrophage foam cell formation, inhibit polarization of macrophages towards the M1 phenotype and promote polarization towards the M2 phenotype, while also ameliorating inflammatory responses. It has significant effects in the prevention and treatment of atherosclerotic cardiovascular and cerebrovascular diseases. Additionally, it demonstrates remarkable therapeutic efficacy against type II diabetes, hypercholesterolemia, hypertriglyceridemia, fatty liver disease, colitis, polycystic ovary syndrome, obesity, and Alzheimer's disease. It also rescues neuronal apoptosis induced by L-glutamate (L-GLU), significantly inhibits apoptosis in neural cells, and affect improves cognitive function in AD model mice.

The applicant declares that this application uses the aforementioned examples to illustrate the detailed process of this application, but this application is not limited to these detailed processes. This does not mean that this application must rely on these detailed processes to be implemented.

Those skilled in the art should understand that any improvements to this application, equivalent substitutions of various raw materials of the product of this application (especially solvents and emulsifiers), additions of auxiliary components, selections of specific methods, etc., all fall within the protection and disclosure scope of this application.

## Claims

1. A 1,4-polyisoprene dispersion system, **characterized by** remains stable in a mammalian gastric acid environment without clot formation of 1,4-polyisoprene, absence of oral toxicity in mammals, and being free from allergens.

2. The 1,4-polyisoprene dispersion system according to claim 1, wherein the 1,4-polyisoprene dispersion system is selected from a 1,4-polyisoprene solution dispersion system and a 1,4-polyisoprene emulsion dispersion system;
wherein the 1,4-polyisoprene solution dispersion system comprises 1,4-polyisoprene and a solvent;
the 1,4-polyisoprene emulsion dispersion system comprises 1,4-polyisoprene, an emulsifier, and water.

3. The 1,4-polyisoprene dispersion system according to claim 1 or 2, wherein the polymerization degree of the 1,4-polyisoprene is ≥6.

4. The 1,4-polyisoprene dispersion system according to claim 2 or 3, wherein the solvent is selected from any one or a combination of at least two of fatty acids, fatty alcohols, ester compounds, or lipid-like compounds;
preferably, the ester compounds are selected from any one or a combination of at least two of monohydric alcohol ester, dihydric alcohol ester or polyhydric alcohol ester;
preferably, the lipid-like compounds are selected from any one or a combination of at least two of phospholipids, glycolipids, sphingolipids, steroidal compounds, or squalene.

5. The 1,4-polyisoprene dispersion system according to any one of claims 2-4, wherein the mass percentage of 1,4-polyisoprene in the 1,4-polyisoprene solution dispersion system is less than or equal to 60 %, and is not 0, preferably 0.2%-20%.

6. The 1,4-polyisoprene dispersion system according to any one of claims 2-5, wherein the emulsifier is selected selected from any one or a combination of at least two of cationic surfactants, anionic surfactants, amphoteric surfactants, or nonionic surfactants;
preferably, the nonionic surfactants are selected from any one or a combination of at least two of polyoxyethylene (20) sorbitan monolaurate, polyoxyethylene (20) sorbitan monopalmitate, polyoxyethylene (20) sorbitan monostearate, polyoxyethylene (20) sorbitan monooleate, decaglycerol monolaurate, decaglycerol monomyristate, decaglycerol monopalmitate, decaglycerol monooleate, decaglycerol monostearate, or C12-C18 monofatty acid sucrose esters.

7. The 1,4-polyisoprene dispersion system according to any one of claims 2-6, wherein the mass percentage of 1,4-polyisoprene in the 1,4-polyisoprene emulsion dispersion system is greater than 0% and not more than 80%, preferably 0.5%-70%;
preferably, the average droplet size of the emulsion in the 1,4-polyisoprene emulsion dispersion system is ≤10 µm.

8. A pharmaceutical active ingredient, comprising the 1,4-polyisoprene dispersion system according to any one of claims 1-7.

9. A pharmaceutical composition, comprising the pharmaceutical active ingredient according to claim 9;
preferably, the pharmaceutical composition comprises an active ingredient for treating chronic metabolic diseases;
preferably, the chronic metabolic diseases are selected from atherosclerotic cardiovascular and cerebrovascular diseases, type II diabetes, hypercholesterolemia, hypertriglyceridemia, fatty liver disease, colitis, polycystic ovary syndrome, and obesity.

10. Use of a pharmaceutical composition in the manufacture of a medicament for the treatment of Alzheimer's disease, wherein the pharmaceutical composition comprises a pharmaceutical active ingredient according to claim 8, and wherein the pharmaceutical active ingredient comprises the 1,4-polyisoprene dispersion system according to any one of claims 1 to 7.
